# EUROPEAN PATENT APPLICATION

(11) **EP 4 413 968 A1**
(43) Date of publication of application: **14.08.2024**
(21) Application number: 22878640.6
(22) Date of filing: 07.10.2022
(51) Int. Cl.: A61K 6/887, A61K 6/30, A61K 6/64, A61K 6/79, A61K 6/836

(54) **CURABLE DENTAL COMPOSITION AND DENTAL KIT**

(30) Priority: 08.10.2021 JP 2021166531
(71) Applicant: Kuraray Noritake Dental Inc., Kurashiki-shi, Okayama 710-0801 (JP)
(72) Inventor: KAWANA, Mariko, Tainai-shi, Niigata 959-2653 (JP)
(74) Representative: D Young & Co LLP
(86) International application number: PCT/JP2022/037737
(87) International publication number: WO 2023/058773

(57) **Abstract**

The present invention provides a dental curable composition that excels in qualities such as working time margin, and bond strength to dentin. The present invention relates to a dental curable composition comprising a polymerizable monomer (A) having an acidic group, a polymerizable monomer (B) having no acidic group, a chemical polymerization initiator (C), a polymerization accelerator (D), a transition metal compound (E), and a ligand compound (F), wherein the ligand compound (F) is at least one compound selected from the group consisting of a ligand containing a phosphorus atom, and a ligand containing a nitrogen atom.

## Description

### TECHNICAL FIELD

The present invention relates to dental curable compositions and dental kits used therewith that are used in applications such as bonding of dental prostheses, such as crowns, inlays, and bridges, to tooth structures, and construction of abutments in dental treatments. Specifically, the invention relates to dental curable compositions and dental kits used therewith that demonstrate excellent chemical curability, bond strength to dentin, and mechanical strength. The invention also relates to a dental kit that comprises a water-based dental adhesive composition (I) or dental adhesive composition (III), along with a dental paste composition (II), and exhibits an adequate working time margin.

### BACKGROUND ART

Adhesive materials and restorative filling materials are widely used for the restorative treatment of tooth defects caused by caries, fractures, or other forms of damage. Resin-based dental curable compositions comprising components such as radical polymerizable monomers, polymerization initiators, and fillers are commonly employed as such adhesive materials and restorative filling materials in tooth restoration.

Within such resin-based dental curable compositions, the material used to bond the prosthesis to the tooth structure is called dental resin cement. In the restorative treatment of deep cavities reaching the pulp, the procedure requires the removal of the pulp and the construction of the abutment tooth. The material utilized for this purpose is referred to as composite resin for dental abutment construction. Both dental resin cement and composite resin for dental abutment construction are paste-form compositions, and are typically produced by mixing a polymerizable monomer-containing liquid composition, containing dissolved components such as polymerizable monomers, a polymerization initiator system, and stabilizers, with components such as powdery fillers. These materials are supplied in containers to dentists for use. It is essential for dental curable compositions to maintain certain performance standards during their designated period of use.

To enhance the bond strength between the paste-form composition and the tooth structure or prosthesis, liquid compositions are occasionally used with the paste-form composition by being applied to the adherend beforehand. Such liquid compositions are known as water-based dental adhesive compositions (primers) and dental adhesive compositions (bonds). Dental curable compositions fall into two categories: self-adhesive compositions capable of bonding to the tooth structure or prostheses by itself, and kits containing liquid compositions, each serving distinct purposes and applications. These dental curable compositions, whether it is a paste or liquid composition, are supplied in a container to the dentist, the end-user.

Generally, (meth)acrylate is used as a polymerizable monomer incorporated in dental cements, dental abutment construction materials, water-based dental adhesive compositions, and dental adhesive compositions. Polymerizable monomers having acidic groups such as phosphoric acid groups or carboxyl groups are also incorporated to confer dental cements with self-adhesive properties to tooth structures or prostheses. Dental resin cements conferred with adhesive properties by the inclusion of polymerizable monomers having an acidic group are called self-adhesive dental resin cements.

In order to polymerize and cure these dental curable compositions through chemical polymerization, a redox-type polymerization initiator system is sometimes used that comprises a chemical polymerization initiator and a polymerization accelerator. In this case, the chemical polymerization initiator and the polymerization accelerator are separately packed within, for example, a first agent containing the chemical polymerization initiator, and a second agent containing the polymerization accelerator. These are supplied to the end-user, the dentist, in the form of a separate-pack type dental curable composition. In using such a separate-pack type dental curable composition, the dentist mixes the first agent and the second agent, containing the chemical polymerization initiator and the polymerization accelerator, respectively, immediately before use. This causes a redox reaction, creating radicals that drive the polymerization and curing of the dental curable composition.

Traditionally, a common choice for the redox-type polymerization initiator system used in dental curable compositions has been a polymerization initiator system that combines benzoyl peroxide and aromatic amine compounds. However, using this initiator system presents an issue of low storage stability in the composition due to the low heat stability of benzoyl peroxide. Specifically, prolonged storage of compositions containing benzoyl peroxide at temperatures higher than room temperature may lead to issues such as reduced curability and premature solidification of the composition before use due to benzoyl peroxide decomposition. Hence, when supplying compositions containing benzoyl peroxide to users like dentists, it is essential to take measures, for example, such as specifying a storage temperature below room temperature or shortening the expiration date. Indeed, there is potential for improvement in terms of usability and quality stability.

Against this backdrop, chemical polymerization initiator systems have come to be used that contain more stable organic peroxides as alternatives to benzoyl peroxide. Specifically, dental curable compositions are available that comprise a redox polymerization initiator combining thiourea derivatives with stable peroxides. Such chemical polymerization initiator systems have high heat stability, and dental curable compositions comprising such systems exhibit high storage stability at room temperature. A problem, however, is low curability. To address this issue, polymerization initiator systems have been developed that incorporate transition metal compounds in the chemical polymerization initiator system (for example, Patent Literatures 1 to 3).

Patent Literature 1 discloses a chemical polymerization initiator system comprising a hydroperoxide, a thiourea derivative, and a copper compound serving as a transition metal compound. This chemical polymerization initiator system is described as having achieved high storage stability and curability.

Patent Literature 2 discloses a chemical polymerization initiator system comprising t-butyl hydroperoxide, a thiourea derivative, and a vanadium compound serving as a transition metal compound. This chemical polymerization initiator system is described as having achieved even higher storage stability.

Compositions comprising high-stability organic peroxides and transition metal compounds are also disclosed in conjunction with kits that include dental curable compositions and pretreatment agents. Patent Literature 3 discloses a kit that includes a paste and a pretreatment agent, with the paste comprising a hydroperoxide, a cyclic thiourea compound, and a vanadium and/or copper compound. This dental curable composition is described as having outstanding adhesive properties, curability, and storage stability.

In Patent Literature 4, a dental curable composition comprising a copper compound along with a benzotriazole compound or benzoimidazole compound is disclosed as a high-stability redox polymerization initiator system, aside from the peroxide and thiourea derivative combination. It is stated that this dental curable composition exhibits excellent curability and low discoloration through the use of the polymerization initiator system that combines the aforementioned compounds.

### CITATION LIST

### Patent Literature

Patent Literature 1: JP 2007-056020 A
Patent Literature 2: JP 2009-144054 A
Patent Literature 3: WO2014/156077
Patent Literature 4: WO2012/086189

### SUMMARY OF INVENTION

### Technical Problem

The chemical polymerization initiator systems described in Patent Literatures 1 and 2 lack assessment regarding both working time and adhesive properties to tooth structure. While high chemical curability provides superior properties including mechanical strength and adhesive properties to tooth structure, it is not always possible to provide a desired working time when the chemical curability is excessively high.

Patent Literatures 3 and 4 demonstrated favorable adhesive properties as dental cement compositions. However, the present inventor found that further improvements are needed concerning curability under altered conditions, including the composition, when various practical aspects, such as applications, are taken into account.

It is accordingly an object of the present invention to provide a dental curable composition that excels in qualities such as working time margin, and bond strength to dentin.

### Solution to Problem

The present inventor conducted intensive studies on dental curable compositions, and found that the activity of the polymerization initiator system can greatly improve when a ligand compound is incorporated in a dental curable composition that comprises a polymerizable monomer having an acidic group, a polymerizable monomer having no acidic group, a chemical polymerization initiator, a polymerization accelerator, and a transition metal compound. This finding, addressing the foregoing issues, led to the completion of the present invention.

Specifically, the present invention provides the following dental curable com positions.
[1] A dental curable composition comprising a polymerizable monomer (A) having an acidic group, a polymerizable monomer (B) having no acidic group, a chemical polymerization initiator (C), a polymerization accelerator (D), a transition metal compound (E), and a ligand compound (F),
   wherein the ligand compound (F) is at least one compound selected from the group consisting of a ligand containing a phosphorus atom, and a ligand containing a nitrogen atom.
[2] The dental curable composition according to [1], wherein:
   the ligand compound (F) is a ligand containing a phosphorus atom, and
   the ligand containing a phosphorus atom is at least one compound selected from the group consisting of a compound represented by the following general formula (1), a compound represented by general formula (2), a compound represented by general formula (3), and a compound represented by general formula (4), 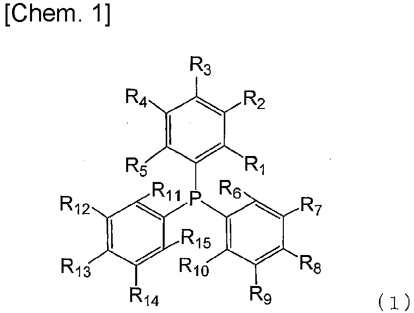 wherein R₁ to R₁₅ each independently represent a hydrogen atom, a halogen atom, a polar group, an optionally substituted alkyl group, or an optionally substituted alkoxy group, 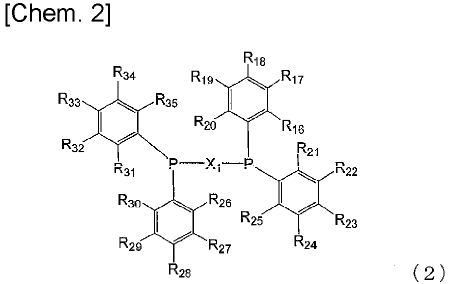 wherein R₁₆ to R₃₅ each independently represent a hydrogen atom, a halogen atom, a polar group, an optionally substituted alkyl group, or an optionally substituted alkoxy group, and X₁ represents an optionally substituted divalent aliphatic group, 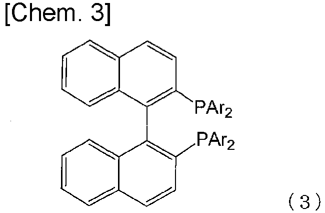 wherein Ar each independently represent a group represented by the following general formula (3-a), 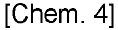 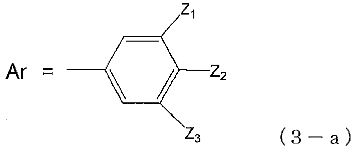 wherein Z₁ to Z₃ are each independently a hydrogen atom, a halogen atom, an optionally substituted alkyl group, or an optionally substituted alkoxy group, and at least one of Z₁ to Z₃ is a hydrogen atom,

      P(OY₁)₃ (4),

      wherein Y₁ each independently represent an optionally substituted alkyl group, or an optionally substituted aryl group.
[3] The dental curable composition according to [2], wherein the ligand containing a phosphorus atom is at least one compound selected from the group consisting of a compound represented by general formula (1), and a compound represented by general formula (2).
[4] The dental curable composition according to [1], wherein:
   the ligand compound (F) is a ligand containing a nitrogen atom,
   the ligand containing a nitrogen atom is at least one compound selected from the group consisting of a compound represented by general formula (5), a compound represented by general formula (6), and a polydentate ligand (7) containing a nitrogen-containing heterocyclic ring, and
   the polydentate ligand (7) is a bidentate or higher dentate ligand compound that comprises a heterocyclic ring containing a five-membered or six-membered ring having a nitrogen atom, and has two or more nitrogen atoms within the molecule,

      R₃₆R₃₇N-X₂-NR₃₈R₃₉ (5),

   wherein R₃₆ to R₃₉ each independently represent an optionally substituted alkyl group, and X₂ represents an optionally substituted divalent aliphatic group, 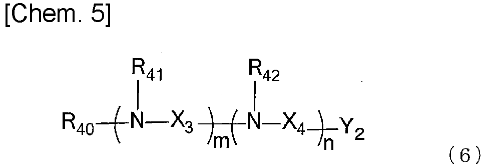
   wherein R₄₀, R₄₁, and R₄₂ each independently represent an optionally substituted alkyl group, X₃ and X₄ each independently represent an optionally substituted divalent aliphatic group that may contain an oxygen atom and/or a nitrogen atom, m and n each independently represent an integer of 1 or more, Y₂ represents an optionally substituted monoalkylamino group or dialkylamino group, and any two or more of R₄₀, R₄₁, R₄₂, and Y₂ may together form a ring, and R₄₁, R₄₂, X₃, and X₄ may be the same or different when R₄₁, R₄₂, X₃, and X₄ are plural.
[5] The dental curable composition according to any one of [1] to [4], wherein the chemical polymerization initiator (C) is at least one selected from the group consisting of a hydroperoxide, a peroxyester, and a peroxydisulfate.
[6] The dental curable composition according to any one of [1] to [5], wherein the polymerization accelerator (D) is at least one selected from the group consisting of a thiourea compound, an aromatic sulfinic acid and a salt thereof, and an ascorbic acid compound.
[7] The dental curable composition according to any one of [1] to [6], wherein the transition metal compound (E) is a copper compound and/or a vanadium compound.
[8] The dental curable composition according to any one of [1] to [7], which further comprises a filler (G).
[9] The dental curable composition according to any one of [1] to [8], which is a two-paste composition comprising a first agent and a second agent.
[10] The dental curable composition according to [9], wherein:
   the first agent comprises the polymerizable monomer (A) having an acidic group, the polymerizable monomer (B) having no acidic group, and the chemical polymerization initiator (C),
   the second agent comprises the polymerizable monomer (B) having no acidic group, and the polymerization accelerator (D), and
   the first agent or the second agent comprises the transition metal compound (E) and the ligand compound (F).
[11] The dental curable composition according to any one of [1] to [10], wherein the content of the ligand compound (F) is 0.01 to 5 parts by mass with respect to total 100 parts by mass of the polymerizable monomer components in the dental curable composition.
[12] The dental curable composition according to any one of [1] to [11], wherein the content of the chemical polymerization initiator (C) and the content of the ligand compound (F) are 1 :0.0001 to 1:10 in terms of a mass ratio of chemical polymerization initiator (C):ligand compound (F).
[13] A dental kit comprising a water-based dental adhesive composition (I) and a dental paste composition (II), wherein:
   the water-based dental adhesive composition (I) comprises a polymerizable monomer (A) having an acidic group, a ligand compound (F), and water, and
   the dental paste composition (II) comprises a polymerizable monomer (B) having no acidic group, a chemical polymerization initiator (C), a polymerization accelerator (D), a transition metal compound (E), and a filler (G).
[14] A dental kit comprising a dental adhesive composition (III) and a dental paste composition (II), wherein:
   the dental adhesive composition (III) comprises a polymerizable monomer (A) having an acidic group, a polymerizable monomer (B) having no acidic group, a ligand compound (F), and a photopolymerization initiator system (H), and
   the dental paste composition (II) comprises a polymerizable monomer (B) having no acidic group, a chemical polymerization initiator (C), a polymerization accelerator (D), a transition metal compound (E), and a filler (G).

### Advantageous Effects of Invention

According to the present invention, a dental curable composition can be provided that excels in working time margin, and bond strength to dentin.

According to the present invention, a dental curable composition can be provided that excels in the mechanical strength of the cured product.

According to the present invention, a dental curable composition can be provided that excels in chemical curability and biological safety.

According to the present invention, a dental curable composition can be provided that excels in mechanical strength durability.

### DESCRIPTION OF EMBODIMENTS

A dental curable composition of the present invention comprises a polymerizable monomer (A) having an acidic group, a polymerizable monomer (B) having no acidic group, a chemical polymerization initiator (C), a polymerization accelerator (D), a transition metal compound (E), and at least one ligand compound (F) of a specific structure. The following describes the components contained in a dental curable composition of the present invention.

While the mechanism by which the ligand compound (F) in the polymerization initiator system of the present invention shows its functions remains unclear, the following speculation has been made.

The ligand compound (F) is believed to potentially activate the catalytic cycle by coordinating to the metal atom of transition metal compound (E).

In a polymerization initiator system that comprises a transition metal compound (E), a catalytic cycle occurs where the metal undergoes oxidation and reduction through different components of the polymerization initiator system, generating radicals that lead to a polymerization reaction. When a ligand compound (F) is added to this polymerization initiator system, the ligand compound (F) coordinates with the metal, facilitating the oxidation reaction and/or reduction reaction, and possibly accelerating the catalytic cycle.

If the ligand compound (F) has an electron-donating group, it can stabilize the metal in a higher oxidation state, accelerating the metal oxidation process. Conversely, if the ligand compound (F) has an electron-withdrawing group, it can stabilize the metal in a lower oxidation state, accelerating the metal reduction process.

In this fashion, the ligand compound (F), regardless of whether it has an electron-donating group or electron-withdrawing group, can activate the catalytic cycle, and potentially accelerate the polymerization reaction.

Accelerating the polymerization reaction through these factors allows for adjustments of working time within the appropriate range by addition of ligand compound (F), without excessively increasing the amount of chemical polymerization initiator (C) and polymerization accelerator (D). Additionally, the increased polymerization conversion rate due to this accelerated polymerization can prevent penetration of water into the bonding interface, ultimately enhancing bond strength.

The polymerizable monomer (A) having an acidic group is an essential component for a dental curable composition of the present invention to exhibit adhesive properties. The polymerizable monomer (A) having an acidic group serves to demineralize tooth structure. The polymerizable monomer (A) having an acidic group is a polymerizable monomer having at least one acidic group such as a phosphoric acid group, a phosphonic acid group, a pyrophosphoric acid group, a thiophosphoric acid group, a carboxylic acid group, and a sulfonic acid group, and at least one polymerizable group such as an acryloyl group, a methacryloyl group, an acrylamide group, and a methacrylamide group. In view of adhesive properties to tooth structure, the polymerizable monomer (A) having an acidic group is preferably a monofunctional polymerizable monomer having any one of an acryloyl group, a methacryloyl group, an acrylamide group, and a methacrylamide group as a polymerizable group. Specific examples of the polymerizable monomer (A) having an acidic group are as follows.

Examples of the polymerizable monomer having a phosphoric acid group include:
monofunctional (meth)acrylate compounds having a phosphoric acid group, such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 2-(meth)acryloyloxyethylphenyl hydrogen phosphate, 2-(meth)acryloyloxyethyl-2-bromoethyl hydrogen phosphate, 2-(meth)acryloyloxyethyl-(4-methoxyphenyl)hydrogen phosphate, and 2-(meth)acryloyloxypropyl-(4-methoxyphenyl)hydrogen phosphate, and acid chlorides, alkali metal salts, ammonium salts, and amine salts of these; and
bifunctional (meth)acrylate compounds having a phosphoric acid group, such as bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, and 1,3-di(meth)acryloyloxypropyl dihydrogen phosphate, and acid chlorides, alkali metal salts, ammonium salts, and amine salts of these.

Examples of the polymerizable monomer having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 1 0-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, ammonium salts, and amine salts of these.

Examples of the polymerizable monomer having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, ammonium salts, and amine salts of these.

Examples of the polymerizable monomer having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyeicosyl dihydrogen thiophosphate, and acid chlorides, alkali metal salts, and ammonium salts of these.

Examples of the polymerizable monomer having a carboxylic acid group include:
(meth)acrylic acid, 4-[2-[(meth)acryloyloxy]ethoxycarbonyl]phthalic acid, 4-(meth)acryloyloxyethyl trimellitic acid, 4-(meth)acryloyloxybutyloxycarbonyl phthalic acid, 4-(meth)acryloyloxyhexyloxycarbonyl phthalic acid, 4-(meth)acryloyloxyoctyloxycarbonyl phthalic acid, 4-(meth)acryloyloxydecyloxycarbonyl phthalic acid, and acid anhydrides of these; and
5-(meth)acryloylaminopentylcarboxylic acid, 6-(meth)acryloyloxyhexane-1,1-dicarboxylic acid, 8-(meth)acryloyloxyoctane-1,1-dicarboxylic acid, 10-(meth)acryloyloxydecane-1,1 -dicarboxylic acid, 11-(meth)acryloyloxyundecane-1,1-dicarboxylic acid, and acid chlorides, alkali metal salts, ammonium salts, and amine salts of these.

Examples of the polymerizable monomer having a sulfonic acid group include 2-(meth)acrylamide-2-methylpropanesulfonic acid, 2-sulfoethyl (meth)acrylate, and acid chlorides, alkali metal salts, ammonium salts, and amine salts of these.

Preferred among these polymerizable monomers (A) having an acidic group are polymerizable monomers having a phosphoric acid group, a polymerizable monomers having a pyrophosphoric acid group, and polymerizable monomers having a carboxylic acid group, particularly preferably polymerizable monomers having a phosphoric acid group, and polymerizable monomers having a carboxylic acid group, because these polymerizable monomers exhibit even superior adhesion to tooth structure. Even more preferred are phosphoric acid group-containing (meth)acrylate monofunctional polymerizable monomers or carboxylic acid group-containing (meth)acrylate polymerizable monomers having a C₆ to C₂₀ alkyl group or C₆ to C₂₀ alkylene group as the backbone within the molecule, more preferably phosphoric acid group-containing (meth)acrylate monofunctional polymerizable monomers having a C₈ to C₁₂ alkylene group as the backbone within the molecule. Particularly preferred are 10-methacryloyloxydecyl dihydrogen phosphate, 4-(meth)acryloyloxyethyl trimellitic acid, and 4-(meth)acryloyloxyethyl trimellitic acid anhydride, most preferably 10-methacryloyloxydecyl dihydrogen phosphate.

The polymerizable monomer (A) having an acidic group may be incorporated alone, or two or more thereof may be incorporated in combination. The content of polymerizable monomer (A) having an acidic group is not particularly limited, as long as the present invention can exhibit its effects. However, in view of even greater adhesion, the content of polymerizable monomer (A) having an acidic group ranges preferably from 1 to 50 parts by mass, more preferably from 2 to 25 parts by mass, even more preferably from 2 to 10 parts by mass in total 100 parts by mass of polymerizable monomer components in a dental curable composition of the present invention.

In this specification, "total 100 parts by mass of polymerizable monomer components in a dental curable composition" means the content of when the total amount of the polymerizable monomers contained in the first and second agents is converted to 100 parts by mass.

In embodiments of dental kits such as the dental kit (Y-1) and dental kit (Y-2) described below, the content of each component is the content in each of water-based dental adhesive composition (I), dental paste composition (II), and dental adhesive composition (III). Accordingly, "total 100 parts by mass of polymerizable monomer components" does not mean 100 parts by mass of the total amount of the polymerizable monomer components in the water-based dental adhesive composition (I) and the polymerizable monomer components in the dental paste composition (II).

The polymerizable monomer (B) having no acidic group is a polymerizable monomer that undergoes polymerization as a result of a radical polymerization reaction driven by a polymerization initiator system. The polymerizable monomers constituting the polymerizable monomer (B) having no acidic group in the present invention are not limited to one kind of polymerizable monomer, and may be two or more kinds of polymerizable monomers.

Preferred examples of the polymerizable monomer (B) having no acidic group include the hydrophilic polymerizable monomer (B-1) and hydrophobic polymerizable monomer (B-2) below.

Hydrophilic polymerizable monomer (B-1) means a polymerizable monomer having a solubility of 10 mass% or more in water at 25°C. Preferably, the hydrophilic polymerizable monomer (B-1) is one having a solubility of 30 mass% or more in water at 25°C, more preferably one that can dissolve in water in any proportion at 25°C.

The hydrophilic polymerizable monomer (B-1) promotes penetration of the components of the dental curable composition into tooth structure. The hydrophilic polymerizable monomer (B-1) itself also penetrates into tooth structure, and adheres to the organic component (collagen) in the tooth structure.

Examples of the hydrophilic polymerizable monomer (B-1) include monofunctional (meth)acrylic acid ester polymerizable monomers such as 2-hydroxyethyl acrylate, 2-hydroxyethyl methacrylate (hereinafter, also referred to by the abbreviation "HEMA"), 3-hydroxypropyl (meth)acrylate, 2-hydroxypropyl (meth)acrylate, 1,3-dihydroxypropyl (meth)acrylate, 2,3-dihydroxypropyl (meth)acrylate, and 2-((meth)acryloyloxy)ethyltrimethylammonium chloride; and bifunctional (meth)acrylic acid ester polymerizable monomers such as polyethylene glycol di(meth)acrylate (with an average of 9 or more moles of oxyethylene group added). Preferred is 2-hydroxyethyl (meth)acrylate.

In this specification, "(meth)acryl" means acryl and methacryl, and the same applies to similar expressions, such as "(meth)acryloyl" and "(meth)acrylate".

Hydrophobic polymerizable monomer (B-2) means a crosslinkable polymerizable monomer having a solubility of less than 10 mass% in water at 25°C.

Examples of the hydrophobic polymerizable monomer (B-2) include aromatic monofunctional polymerizable monomers and bifunctional polymerizable monomers, aliphatic monofunctional polymerizable monomers and bifunctional polymerizable monomers, and tri- and higher-functional polymerizable monomers. The hydrophobic polymerizable monomer (B-2) improves properties such as the mechanical strength of cured products of the dental curable composition, and ease of handling.

Examples of the aromatic monofunctional polymerizable monomers include benzyl (meth)acrylate, p-cumyl-phenoxyethylene glycol (meth)acrylate, and 2-phenoxybenzyl (meth)acrylate. Preferred among these are benzyl methacrylate, and p-cumyl-phenoxyethylene glycol methacrylate.

Examples of the aromatic bifunctional polymerizable monomers include aromatic di(meth)acrylates. Specific examples of the aromatic bifunctional polymerizable monomers include 2,2-bis((meth)acryloyloxyphenyl)propane, 2,2-bis[4-(3-acryloyloxy-2-hydroxypropoxy)phenyl]propane, 2,2-bis[4-(2-hydroxy-3-methacryloyloxypropoxy)phenyl]propane (hereinafter, also referred to by the abbreviation "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. Preferred among these are 2,2-bis[4-(3-methacryloyloxy-2-hydroxypropoxy)phenyl]propane, and 2,2-bis(4-methacryloyloxypolyethoxyphenyl)propane (with an average of 2.6 moles of ethoxy group added; hereinafter, also referred to by the abbreviation "D2.6E").

Examples of the aliphatic monofunctional polymerizable monomers include methyl (meth)acrylate, ethyl (meth)acrylate, butyl (meth)acrylate, isobornyl (meth)acrylate, stearyl (meth)acrylate, dicyclopentanyl (meth)acrylate, butoxydiethylene glycol (meth)acrylate, and methoxypolyethylene glycol (meth)acrylate. Preferred among these is isobornyl methacrylate.

Examples of the aliphatic bifunctional polymerizable monomers include:
bifunctional (meth)acrylic acid ester polymerizable monomers such as erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol diacrylate, triethylene glycol dimethacrylate (hereinafter, also referred to by the abbreviation "TEGDMA"), propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)dimethacrylate, and 1,2-bis(3-methacryloyloxy-2-hydroxypropoxy)ethane; and
(meth)acrylamide polymerizable monomers such as N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

Preferred among these are glycerol dimethacrylate, triethylene glycol di(meth)acrylate, neopentyl glycol dimethacrylate, 2,2,4-trimethylhexamethylenebis(2-carbamoyloxyethyl)dimethacrylate, and 1,2-bis(3-methacryloyloxy-2-hydroxypropyloxy)ethane.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacryl ate, and 1,7-diacryloyloxy-2,2,6,6-tetraacryloyloxymethyl-4-oxaheptane.

In view of the bond strength and polymerization curability of a dental curable composition of the present invention, more preferred among these polymerizable monomers (B) having no acidic group are HEMA, Bis-GMA, D2.6E, and TEGDMA.

The polymerizable monomer (B) having no acidic group (hydrophilic polymerizable monomer (B-1), hydrophobic polymerizable monomer (B-2)) may be incorporated alone, or two or more thereof may be incorporated in combination.

The content of polymerizable monomer (B) having no acidic group is not particularly limited, as long as the present invention can exhibit its effects. However, in view of providing a composition having high penetrability into the tooth structure and superior adhesive properties, as well as providing a sufficient mechanical strength, the content of polymerizable monomer (B) having no acidic group ranges preferably from 50 to 99 parts by mass, more preferably from 60 to 98 parts by mass, even more preferably from 70 to 95 parts by mass in total 100 parts by mass of polymerizable monomer components in the dental curable composition.

In view of superior adhesive properties to tooth structure and mechanical strength, as well as outstanding mechanical strength durability when combined with the ligand compound (F) or other components, it is preferable in certain embodiments that the content of hydrophilic polymerizable monomer (B-1) ranges preferably from 0 to 30 parts by mass, more preferably from 0 to 20 parts by mass, even more preferably from 0 to 15 parts by mass in total 100 parts by mass of polymerizable monomer components in the dental curable composition.

The following describes the polymerization initiator system.

The polymerization initiator system in a dental curable composition of the present invention comprises a chemical polymerization initiator (C), a polymerization accelerator (D), a transition metal compound (E), and at least one ligand compound (F) selected from specific structures. The chemical polymerization initiator (C) and the polymerization accelerator (D) are individually contained in separate packaging forms, and need to be mixed immediately before use.

Examples of the chemical polymerization initiator (C) include organic peroxides and inorganic peroxides. However, the chemical polymerization initiator (C) is not limited to these, and may be a known chemical polymerization initiator.

The chemical polymerization initiator (C) may be incorporated alone, or two or more thereof may be incorporated in combination.

Typical examples of the organic peroxides include hydroperoxides, peroxyesters, ketone peroxides, peroxyketals, dialkyl peroxides, diacyl peroxides, and peroxydicarbonates.

Specific examples of hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide (hereinafter, also referred to by the abbreviation "THP").

The peroxyesters may be any known peroxyesters, provided that the peroxy group (-OO- group) has an acyl group at one end, and a hydrocarbon group (or a similar group) at the other end. Specific examples include α,α-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethylperoxy-2-ethylhexanoate, t-hexyl peroxy-2-ethylhexanoate, t-butyl peroxy-2-ethylhexanoate, t-butyl peroxyisobutyrate, t-hexyl peroxyisopropyl monocarbonate, t-butyl peroxymaleic acid, t-butyl peroxy-3,5,5-trimethylhexanoate, t-butyl peroxylaurate, 2,5-dimethyl-2,5-bis(m-toluoylperoxy)hexane, t-butyl peroxyisopropyl monocarbonate, t-butyl peroxy-2-ethylhexyl monocarbonate, t-hexyl peroxybenzoate, 2,5-dimethyl-2,5-bis(benzoylperoxy)hexane, t-butyl peroxyacetate, t-butyl peroxy-m-toluoylbenzoate, t-butyl peroxybenzoate (hereinafter, also referred to by the abbreviation "BPB"), and bis(t-butylperoxy)isophthalate. These may be used alone, or two or more thereof may be used in appropriate combinations. In view of storage stability and reactivity, preferred among these are t-butyl peroxymaleic acid, t-butyl peroxy-3,5,5-trimethylhexanoate, t-butyl peroxybenzoate, t-butyl peroxyisopropyl monocarbonate, t-butyl peroxy-2-ethylhexyl monocarbonate, and t-butyl peroxyacetate, more preferably t-butyl peroxybenzoate.

Examples of the ketone peroxides include methyl ethyl ketone peroxide, cyclohexanone peroxide, methyl cyclohexanone peroxide, methyl acetoacetate peroxide, and acetyl acetone peroxide.

Examples of the peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl-4,4-bis(t-butylperoxy)valerate, and 2,2-bis(4,4-di-t-butylperoxycyclohexyl)propane.

Examples of the dialkyl peroxides include α,α-bis(t-butylperoxy)diisopropylbenzene, dicumyl peroxide, 2,5-dimethyl-2,5-bis(t-butylperoxy)hexane, t-butyl cumylperoxide, di-t-butyl peroxide, and 2,5-dimethyl-2,5-bis(t-butylperoxy)3-hexyne.

Examples of the diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of the peroxydicarbonates include di-n-propyl peroxydicarbonate, diisopropyl peroxydicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of the inorganic peroxides include peroxydisulfates and peroxydiphosphates. In view of curability, peroxydisulfates are preferred.

Specific examples of peroxydisulfates include sodium peroxydisulfate, potassium peroxydisulfate (hereinafter, also referred to by the abbreviation "KPS"), aluminum peroxydisulfate, and ammonium peroxydisulfate.

A certain embodiment is, for example, a dental curable composition in which the chemical polymerization initiator (C) is at least one selected from the group consisting of a hydroperoxide, a peroxyester, and a peroxydisulfate.

In view of curability, the content of chemical polymerization initiator (C) is preferably 0.01 to 10 parts by mass, more preferably 0.01 to 8 parts by mass, even more preferably 0.02 to 5 parts by mass with respect to total 100 parts by mass of polymerizable monomer components in a dental curable composition of the present invention.

Examples of the polymerization accelerator (D) include aromatic amines, aromatic sulfinic acids and salts thereof, sulfur-containing reducing inorganic compounds, thiourea compounds, ascorbic acid compounds, borate compounds, barbituric acid compounds, benzotriazole compounds, and benzoimidazole compounds. The polymerization accelerator (D) may be used alone, or two or more thereof may be used in combination.

A certain embodiment is, for example, a dental curable composition in which the polymerization accelerator (D) is at least one selected from the group consisting of a thiourea compound, an aromatic sulfinic acid or salts thereof, and an ascorbic acid compound.

The aromatic amines may be known aromatic amines such as aromatic secondary amines or aromatic tertiary amines, preferably aromatic tertiary amines with no electron-withdrawing group in the aromatic ring.

Examples of aromatic tertiary amines with no electron-withdrawing group in the aromatic ring include N,N-bis(2-hydroxyethyl)-3,5-dimethylaniline, N,N-bis(2-hydroxyethyl)-p-toluidine (hereinafter, also referred to by the abbreviation "DEPT"), N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline. In view of redox reactivity, preferred among these is N,N-bis(2-hydroxyethyl)-p-toluidine.

The content of aromatic amines is preferably 0.01 to 5 parts by mass, more preferably 0.05 to 4 parts by mass, even more preferably 0.1 to 3 parts by mass with respect to total 100 parts by mass of polymerizable monomer components in a dental curable composition of the present invention. With an aromatic amine content of 0.01 parts or more by mass, the resulting cured product can have superior mechanical strength. With an aromatic amine content of 5 parts or less by mass, it is possible to reduce discoloration in the cured product of the resultant dental curable composition in water.

Examples of the aromatic sulfinic acids and salts thereof include lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of compounds such as benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid (hereinafter, sodium salts are also referred to by the abbreviation "TPSS"), p-chlorobenzenesulfinic acid, and naphthalenesulfinic acid. In view of the curability and storage stability of the dental curable composition obtained, preferred are lithium salts, sodium salts, potassium salts, magnesium salts, and calcium salts of 2,4,6-trimethylbenzenesulfinic acid and 2,4,6-triisopropylbenzenesulfinic acid, more preferably lithium salts, sodium salts, potassium salts, magnesium salts, and calcium salts of 2,4,6-triisopropylbenzenesulfinic acid.

The content of aromatic sulfinic acids and salts thereof is preferably 0.01 to 5 parts by mass, more preferably 0.05 to 4 parts by mass, even more preferably 0.1 to 3 parts by mass with respect to total 100 parts by mass of polymerizable monomer components in a dental curable composition of the present invention. When the content of aromatic sulfinic acids and salts thereof is 0.01 parts or more by mass and 5 parts or less by mass, the cured product of the resultant dental curable composition can have superior mechanical strength.

Examples of the sulfur-containing reducing inorganic compounds include sulfites, bisulfites, pyrosulfites, thiosulfates, thionates, and dithionites. Preferred among these are sulfites and bisulfites.

Specific examples of the sulfur-containing reducing inorganic compounds include sodium sulfite, potassium sulfite, calcium sulfite, ammonium sulfite, sodium bisulfite, and potassium bisulfite.

The content of the reducing inorganic compound is preferably 0.01 to 15 parts by mass, more preferably 0.05 to 10 parts by mass, even more preferably 0.1 to 5 parts by mass with respect to total 100 parts by mass of polymerizable monomer components in a dental curable composition of the present invention. When the content of the reducing inorganic compound is 0.01 parts or more by mass, it is possible to reduce a decrease in the bond strength of the resultant dental curable composition to moist materials such as tooth structure. When the content of the reducing inorganic compound is 15 parts or less by mass, there is no possibility of decreasing the mechanical strength in the cured product of the resultant dental curable composition.

Examples of the thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea (hereinafter, also referred to by the abbreviation "PyTU"), 4,4-dimethylethylenethiourea, phenylthiourea, N-(2-hydroxyphenyl)thiourea, N-(2,6-dihydroxyphenyl)thiourea, N-(2,4-dihydroxyphenyl)thiourea, N-(2-hydroxy-4-methoxyphenyl)thiourea, N-(2-hydroxy-4-ethoxyphenyl)thiourea, N-(2-hydroxy-4-dimethylaminophenyl)thiourea, N-(2-hydroxy-4-diethylaminophenyl)thiourea, N-(2-methoxyphenyl)thiourea, N-(2-ethoxyphenyl)thiourea, N-(2,6-dimethoxyphenyl)thiourea, N-(2,4-dimethoxyphenyl)thiourea, N-(2-methoxy-4-hydroxyphenyl)thiourea, N-(2-methoxy-4-dimethylaminophenyl)thiourea, N-(2-methoxy-3-hydroxyphenyl)thiourea, N-(2-dimethylaminophenyl)thiourea, N-(2-diethylaminophenyl)thiourea, N-(2-dimethylamino-4-methoxyphenyl)thiourea, N-(2-dimethylamino-4-hydroxyphenyl)thiourea, N-(2-aminophenyl)thiourea, N-(2,4-diaminophenyl)thiourea, N-(2,6-diaminophenyl)thiourea, and N-(2-amino-4-hydroxyphenyl)thiourea.

The content of thiourea compounds is preferably 0.01 to 15 parts by mass, more preferably 0.05 to 10 parts by mass, even more preferably 0.1 to 5 parts by mass with respect to total 100 parts by mass of polymerizable monomer components in a dental curable composition of the present invention. When the content of thiourea compounds is 0.01 parts or more by mass, it is possible to reduce a decrease in the bond strength of the resultant dental curable composition to moist materials such as tooth structure. When the content of thiourea compounds is 15 parts or less by mass, the cured product of the resultant dental curable composition can have superior mechanical strength.

Examples of the ascorbic acid compounds include salts, esters, and ethers of ascorbic acid. Preferred among these are salts and esters of ascorbic acid.

Examples of salts of ascorbic acid include sodium L-ascorbate, calcium L-ascorbate, potassium ascorbate, and stereoisomers of these (for example, sodium isoascorbate). Preferred among these is sodium L-ascorbate.

Examples of esters of ascorbic acid include those formed by a reaction of carboxylic acid with one or more of the hydroxyl groups of ascorbic acid.

Preferred examples of carboxylic acids include fatty acids such as C6 to C30 saturated fatty acids or unsaturated fatty acids, for example, caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. The fatty acids have preferably 10 to 28 carbon atoms, more preferably 12 to 26 carbon atoms, even more preferably 14 to 24 carbon atoms. Particularly preferred among these are esters of stearic acid and ascorbic acid, and esters of palmitic acid and ascorbic acid (ascorbyl palmitate).

Examples of ethers of ascorbic acid include ethyl ascorbic ether, and cetyl ascorbic ether.

In view of curability, the mechanical strength of the cured product, and adhesive properties to tooth structure, the content of ascorbic acid compounds ranges preferably from 0.01 to 8 parts by mass, more preferably from 0.1 to 5 parts by mass, even more preferably from 0.5 to 2 parts by mass with respect to total 100 parts by mass of polymerizable monomer components in the dental curable composition.

Examples of the borate compounds include aryl borate compounds having 1 to 4 aryl groups per molecule (for example, tetraphenylboron, tetrakis(p-chlorophenyl)boron), and salts of these.

Examples of the barbituric acid compounds include barbituric acid, 5-butylbarbituric acid, 1 ,3,5-trimethylbarbituric acid, 1-cyclohexyl-5-ethylbarbituric acid, 1-benzyl-5-phenylbarbituric acid, and salts of these.

Examples of the benzotriazole compounds and benzoimidazole compounds include compounds represented by the following general formulae (8) and (9).

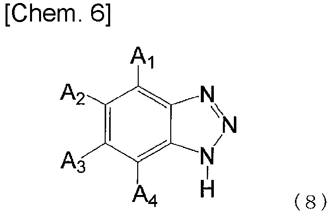

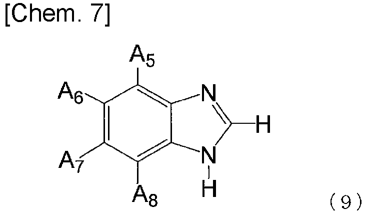

In these general formulae, A₁ to A₈ each independently represent a hydrogen atom, a hydroxyl group, an alkyl group, aryl group, an alkoxy group, an alkenyl group, an aralkyl group, or a halogen atom.

The alkyl groups represented by A₁ to A₈ may be linear, branched, or cyclic, and are preferably ones with 1 to 10 carbon atoms. Examples of the alkyl groups include methyl groups, ethyl groups, n-propyl groups, isopropyl groups, cyclopropyl groups, n-butyl groups, isobutyl groups, sec-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups. Particularly preferred among these are methyl groups and ethyl groups.

The aryl groups represented by A₁ to A₈ are preferably ones with 6 to 14 carbon atoms. Examples of the aryl groups include phenyl groups, naphthyl groups, and anthryl groups.

The alkoxy groups represented by A₁ to A₈ may be linear, branched, or cyclic, and are preferably ones with 1 to 8 carbon atoms. Examples of the alkoxy groups include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkenyl groups represented by A₁ to A₈ may be linear, branched, or cyclic, and are preferably ones with 2 to 6 carbon atoms. Examples of the alkenyl groups include vinyl groups, allyl groups, methylvinyl groups, propenyl groups, butenyl groups, pentenyl groups, hexenyl groups, cyclopropenyl groups, cyclobutenyl groups, cyclopentenyl groups, and cyclohexenyl groups.

Examples of the aralkyl groups represented by A₁ to A₈ include alkyl groups (particularly, C1 to C10 alkyl groups) substituted with aryl groups (particularly, C6 to C10 aryl groups). Specific examples include benzyl groups.

Examples of the halogen atoms represented by A₁ to A₈ include a fluorine atom, a chlorine atom, a bromine atom, and an iodine atom. Preferred as A₁ to A₈ are hydrogen atoms or methyl groups.

The benzotriazole compounds and benzoimidazole compounds may be used alone, or two or more thereof may be used in combination.

Specific examples of the benzotriazole compounds and benzoimidazole compounds include 1H-benzotriazole, 5-methyl-1H-benzotriazole, 5,6-dimethyl-1H-benzotriazole, benzoimidazole, 5-methylbenzoimidazole, and 5,6-dimethylbenzoimidazole. In view of the shade and storage stability of the composition, preferred are 1H-benzotriazole, and 5-methyl-1H-benzotriazole.

In view of the mechanical strength of the cured product, and adhesive properties to tooth structure, the content of benzotriazole compounds and benzoimidazole compounds is preferably 0.01 to 10 parts by mass, more preferably 0.05 to 5 parts by mass, even more preferably 0.5 to 3 parts by mass with respect to total 100 parts by mass of polymerizable monomer components in the dental curable composition.

In view of the mechanical strength of the cured product, and adhesive properties to tooth structure, the content of polymerization accelerator (D) is preferably 0.001 to 15 parts by mass, more preferably 0.01 to 12 parts by mass, even more preferably 0.05 to 10 parts by mass in terms of a total amount in the dental curable composition, relative to total 100 parts by mass of polymerizable monomer components in the dental curable composition.

In view of achieving a reduced content for the chemical polymerization initiator system (chemical polymerization initiator (C), polymerization accelerator (D)) while providing a superior mechanical strength in the cured product, and excellent adhesive properties to tooth structure, it is preferable in certain preferred embodiments that the content of polymerization accelerator (D) be 0.01 to 5 parts by mass, more preferably 0.02 to 3 parts by mass, even more preferably 0.03 to 2 parts by mass with respect to total 100 parts by mass of polymerizable monomer components in the dental curable composition. If necessary, the upper limit may be less than 1 part by mass, or less than 0.5 parts by mass.

Preferred for use as transition metal compound (E) are copper compounds and/or vanadium compounds. The transition metal compound (E) may be used alone, or two or more thereof may be used in combination.

Examples of the copper compounds include copper(II) carboxylates, copper(II) β-diketones, copper(II) β-ketoesters, copper alkoxides, copper dithiocarbamates, and salts of copper and inorganic acids.

Examples of the copper(II) carboxylates include copper(II) citrate, copper(II) acetate, copper(II) phthalate, copper(II) tartarate, copper(II) oleate, copper(II)octylate, copper(II) octenate, copper(II) naphthenate, copper(II) methacrylate, and copper(II) 4-cyclohexylbutyrate.

Examples of the copper(II) β-diketones include copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate.

Examples of the copper(II) β-ketoesters include copper(II) ethylacetoacetate.

Examples of the copper alkoxides include copper(II) methoxide, copper(II) ethoxide, copper(II) isopropoxide, copper(II) 2-(2-butoxyethoxy)ethoxide, and copper(II) 2-(2-methoxyethoxy)ethoxide.

Examples of the copper dithiocarbamates include copper(II) dimethyldithiocarbamate.

Examples of the salts of copper and inorganic acids include copper(II) nitrate, copper(II) bromide, and copper(II) chloride.

These may be used alone, or two or more thereof may be used in appropriate combinations. In view of solubility and reactivity to the polymerizable monomers, preferred are copper(II) carboxylates, copper(II) β-diketones, and copper(II) β-ketoesters, particularly preferably copper(II) acetate, and copper(II) acetylacetonate.

Preferred as the vanadium compounds are vanadium compounds with a valency of IV and/or V.

Examples of the vanadium compounds with a valency of IV and/or V include compounds noted in JP 2003-96122 A, including, for example, vanadium(IV) oxide, vanadyl(IV) acetylacetonate, vanadyl oxalate(IV), vanadyl(IV) sulfate, oxobis(1-phenyl-1,3-butanedionate)vanadium(IV), bis(maltolato)oxovanadium(IV), vanadium(V) oxide, sodium metavanadate, and ammonium metavanadate(V). These may be used alone, or two or more thereof may be used in appropriate combinations.

In view of curability, the mechanical strength of the cured product, and adhesive properties to tooth structure, the content of the transition metal compound (E) ranges preferably from 0.0001 to 1 parts by mass, more preferably from 0.0002 to 0.5 parts by mass, even more preferably from 0.0003 to 0.2 parts by mass with respect to total 100 parts by mass of polymerizable monomer components in the dental curable composition.

The transition metal compound (E) generally tends to become more toxic with increased intake into the human body. In view of prioritizing biological safety while additionally satisfying curability, the mechanical strength of the cured product, and adhesive properties to tooth structure, it is preferable in certain preferred embodiments that the content of transition metal compound (E) range preferably from 0.0001 parts or more by mass to less than 0.1 parts by mass, more preferably from 0.0002 parts or more by mass to 0.08 parts or less by mass, even more preferably from 0.0003 parts or more by mass to less than 0.05 parts by mass with respect to total 100 parts by mass of polymerizable monomer components in the dental curable composition.

Some transition metal elements utilized in chemical polymerization initiator systems are known as biologically essential trace elements. As a rule, such transition metal elements also become increasingly more toxic with increased intake into the human body.

The chemical polymerization initiator (C) and polymerization accelerator (D) contribute to the generation of radicals during the redox reaction. However, if present in excess, the chemical polymerization initiator (C) and polymerization accelerator (D) may elute into saliva and other bodily fluids within the patient's oral cavity over an extended time period, potentially leading to a decrease in the mechanical strength of the cured product, or a decline in biological safety. That is, a decrease in biological safety may occur depending on the content of the transition metal compound (E) in the dental curable composition.

While the chemical polymerization initiator systems of related art indeed provide outstanding storage stability and curability, the desired performance cannot be achieved unless the content of the chemical polymerization initiator system (chemical polymerization initiator and polymerization accelerator) is increased. In view of minimizing the content of chemical polymerization initiator system, the related art therefore fails to achieve desired effects, such as working time margin and adhesive properties to tooth structure, when the content of transition metal compound (E) is low.

Moreover, many types of transition metal compounds in transition metal compound (E) induce discoloration within the composition by forming black sulfides when they react with sulfides generated by oral bacteria.

As discussed above, it is preferable to decrease the content of transition metal compound (E) to a level that is acceptable in terms of biological safety and aesthetics.

By combining the ligand compound (F) of the present invention with the chemical polymerization initiator system (chemical polymerization initiator (C), polymerization accelerator (D), transition metal compound (E)), the dental curable composition exhibits enhanced effectiveness, such as adhesive properties to tooth structure, compared to dental curable compositions containing no ligand compound (F), even with a reduced content of transition metal compound (E) compared to related art.

From these perspectives, it is preferable in a dental curable composition of the present invention that the content of transition metal compound (E) and the content of ligand compound (F) be 1:5 to 1:10000 in terms of a mass ratio of transition metal compound (E):ligand compound (F). In view of achieving even superior curability, enhanced mechanical strength of the cured product, and greater adhesive properties to tooth structure by facilitating coordination to the metal atom of the transition metal compound (E) and more strongly activating the catalytic cycle, the mass ratio of transition metal compound (E):ligand compound (F) is more preferably 1:15 to 1:7500, even more preferably 1:20 to 1:7000.

A dental curable composition of the present invention comprises a ligand compound (F), and the ligand compound (F) is at least one compound selected from the group consisting of a ligand containing a phosphorus atom, and a ligand containing a nitrogen atom.

The ligand containing a phosphorus atom contains a phosphorus atom as a coordinating atom. The ligand containing a nitrogen atom contains a nitrogen atom as a coordinating atom.

The ligand compound (F) may be a combination of a ligand containing a phosphorus atom, and a ligand containing a nitrogen atom. As described above, the dental curable composition comprises a ligand compound (F), a compound that coordinates with the metal atom of the transition metal compound (E). This allows for the integration of the ligand compound (F) into the chemical polymerization initiator system, enabling a significant enhancement in the activity of the chemical polymerization initiator system.

The effect on the chemical polymerization initiator system due to the inclusion of the ligand compound (F) is believed to remain consistent regardless of the type of polymerizable monomer, and there appear to be no situations where the effect is impeded by the polymerizable monomer, allowing the same effect to be attained across different dental applications. The inclusion of the ligand compound (F) in the dental curable composition also provides excellent durability in mechanical strength.

A certain preferred embodiment is, for example, a dental curable composition in which the ligand compound (F) comprises a ligand containing a nitrogen atom.

Another preferred embodiment is, for example, a dental curable composition in which the ligand compound (F) comprises a ligand containing a phosphorus atom.

Examples of the ligand containing a phosphorus atom include phosphine ligands and phosphite ligands.

Specific examples of the ligand containing a phosphorus atom include compounds represented by the following general formula (1), compounds represented by general formula (2), compounds represented by general formula (3), and compounds represented by general formula (4). These ligands are highly stable in the presence of oxygen, and are not constrained by storage conditions, enabling an even greater enhancement in the activity of the chemical polymerization initiator system.

The ligand containing a phosphorus atom may be used alone, or two or more thereof may be used in combination. 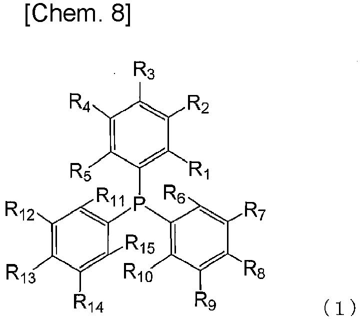 R₁ to R₁₅ each independently represent a hydrogen atom, a halogen atom, a polar group, an optionally substituted alkyl group, or an optionally substituted alkoxy group. 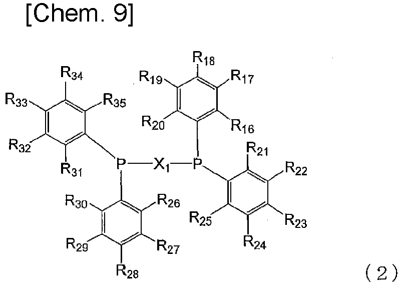 R₁₆ to R₃₅ each independently represent a hydrogen atom, a halogen atom, a polar group, an optionally substituted alkyl group, or an optionally substituted alkoxy group, and X₁ represents an optionally substituted divalent aliphatic group. 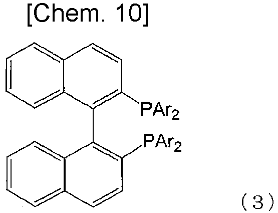 Ar each independently represent a group represented by the following general formula (3-a)). 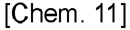 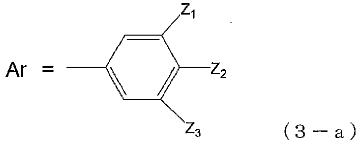 Z₁ to Z₃ each independently represent a hydrogen atom, a halogen atom, an optionally substituted alkyl group, or an optionally substituted alkoxy group, and at least one of Z₁ to Z₃ is a hydrogen atom.

P(OY₁)₃ (4)

Y₁ each independently represent an optionally substituted alkyl group, or an optionally substituted aryl group.

In view of curability, the mechanical strength of the cured product, and adhesive properties to tooth structure, preferred among these ligands containing a phosphorus atom are compounds represented by the general formula (1), and compounds represented by general formula (2).

R₁ to R₁₅ are more preferably hydrogen atoms, optionally substituted alkyl groups, or optionally substituted alkoxy groups.

R₁₆ to R₃₅ are more preferably hydrogen atoms, optionally substituted alkyl groups, or optionally substituted alkoxy groups.

Z₁ to Z₃ are more preferably hydrogen atoms, optionally substituted alkyl groups, or optionally substituted alkoxy groups.

The optionally substituted alkyl groups represented by R₁ to R₁₅ may be linear or branched. The number of carbon atoms in the alkyl groups represented by R₁ to R₁₅ is not particularly limited, and is preferably 1 to 12, more preferably 1 to 6, even more preferably 1 to 4, particularly preferably 1 to 3.

Examples of the alkyl groups represented by R₁ to R₁₅ include methyl groups, ethyl groups, n-propyl groups, isopropyl groups, n-butyl groups, isobutyl groups, sec-butyl groups, tert-butyl groups, n-pentyl groups, isopentyl groups, sec-pentyl groups, neopentyl groups, n-hexyl groups, isohexyl groups, n-heptyl groups, n-octyl groups, n-nonyl groups, n-decyl groups, n-undecyl groups, and n-dodecyl groups. The alkyl groups represented by R₁ to R₁₅ may be unsubstituted.

Examples of the substituents on the alkyl groups represented by R₁ to R₁₅ include halogen atoms (a fluorine atom, a chlorine atom, a bromine atom, an iodine atom), hydroxyl groups, alkoxy groups having 1 to 6 carbon atoms, dialkylamino groups having 1 to 6 carbon atoms in each alkyl group, and amino groups.

Examples of the halogen atoms represented by R₁ to R₁₅ include a fluorine atom, a chlorine atom, a bromine atom, and an iodine atom.

Examples of the polar groups represented by R₁ to R₁₅ include acid anhydride groups, carboxylic acid groups, carboxylic acid ester groups, carboxylic acid chloride groups, carboxylic acid amide groups, carboxylate groups, sulfonic acid groups, sulfonic acid ester groups, sulfonyl chloride groups, sulfonic acid amide groups, sulfonate groups, aldehyde groups, epoxy groups, cyano groups, amino groups, monoalkyl-substituted amino groups, dialkyl-substituted amino groups, imide groups, and oxazoline groups. In view of curability and the mechanical strength of the cured product, preferred are carboxylic acid groups, carboxylic acid ester groups, carboxylic acid chloride groups, carboxylic acid amide groups, carboxylate groups, sulfonic acid groups, sulfonic acid ester groups, sulfonyl chloride groups, sulfonic acid amide groups, sulfonate groups, and aldehyde groups, more preferably carboxylic acid groups, carboxylic acid ester groups, carboxylic acid chloride groups, carboxylate groups, sulfonic acid groups, sulfonic acid ester groups, sulfonyl chloride groups, sulfonate groups, and aldehyde groups, even more preferably carboxylic acid groups, carboxylic acid ester groups, carboxylic acid chloride groups, carboxylate groups, sulfonic acid groups, sulfonic acid ester groups, sulfonyl chloride groups, and sulfonate groups.

Examples of salts of carboxylate groups and sulfonate groups include alkali metal salts such as lithium salts, sodium salts, and potassium salts; and alkali-earth metal salts such as magnesium salts, calcium salts, strontium salts, barium salts, and radium salts. When R₁ to R₁₅ are polar groups, the number of polar groups is preferably 1 to 9, more preferably 1 to 5, even more preferably 1 to 3.

When R₁ to R₁₅ are alkyl groups having a substituent, trifluoromethyl groups represent a specific example of such alkyl groups.

The optionally substituted alkoxy groups represented by R₁ to R₁₅ may be linear or branched. The number of carbon atoms in the alkoxy groups represented by R₁ to R₁₅ is not particularly limited, and is preferably 1 to 12, more preferably 1 to 6, even more preferably 1 to 4, particularly preferably 1 to 3. Examples of the alkoxy groups represented by R₁ to R₁₅ include methoxy groups, ethoxy groups, n-propoxy groups, isopropoxy groups, n-butoxy groups, sec-butoxy groups, tert-butoxy groups, n-pentyloxy groups, isopentyloxy groups, sec-pentyloxy groups, tert-pentyloxy groups, neopentyloxy groups, n-hexyloxy groups, isohexyloxy groups, sec-hexyloxy groups, tert-hexyloxy groups, and neohexyloxy groups. Examples of the substituents on the alkoxy groups represented by R₁ to R₁₅ include the same substituents as those of the alkyl groups represented by R₁ to R₁₅.

R₁ to R₁₅ may be the same or different. For example, R₁ to R₁₅ may partly represent the same hydrogen atoms, alkyl groups, or alkoxy groups.

The optionally substituted alkyl groups represented by R₁₆ to R₃₅ are the same as the optionally substituted alkyl groups represented by R₁ to R₁₅.

The optionally substituted alkoxy groups represented by R₁₆ to R₃₅ are the same as the optionally substituted alkoxy groups represented by R₁ to R₁₅.

The halogen atoms represented by R₁₆ to R₃₅ are the same as the halogen atoms represented by R₁ to R₁₅.

The polar groups represented by R₁₆ to R₃₅ are the same as the polar groups represented by R₁ to R₁₅.

The optionally substituted divalent aliphatic group represented by X₁ may be linear or branched. The divalent aliphatic group has preferably 1 to 20 carbon atoms, more preferably 1 to 16 carbon atoms, even more preferably 1 to 12 carbon atoms, particularly preferably 1 to 8 carbon atoms.

Examples of the divalent aliphatic group include alkylene groups, alkenylene groups, and alkynylene groups. Preferred are alkylene groups. Examples of the alkylene groups include methylene groups, ethylene groups, propylene groups, butylene groups, methylpropylene groups, dimethylpropylene groups, pentamethylene groups, hexamethylene groups, heptamethylene groups, octamethylene groups, nonamethylene groups, decamethylene groups, undecamethylene groups, and dodecamethylene groups.

Examples of the substituents on the divalent aliphatic group represented by X₁ include the same substituents as those of the alkyl groups represented by R₁ to R₁₅.

In formula (3), the multiple Ar may be the same or different. In groups represented by general formula (3-a), Z₁ to Z₃ may be the same or different. The optionally substituted alkyl groups represented by Z₁ to Z₃ are the same as the optionally substituted alkyl groups represented by R₁ to R₁₅. At least one of Z₁ to Z₃ is a hydrogen atom, and Z₁ to Z₃ may all be hydrogen atoms. Specific examples of Ar include the following groups.

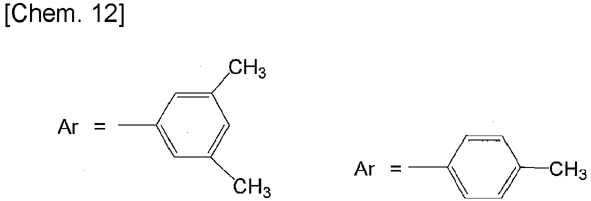

In certain embodiments, one or two of Z₁ to Z₃ are hydrogen atoms, and the remaining one or two of Z₁ to Z₃ may be a linear or branched C1 to C6 alkyl group(s) substituted with a halogen atom, a linear or branched C1 to C4 alkyl group(s) substituted with a fluorine atom, or a trifluoromethyl group. The optionally substituted alkoxy groups represented by Z₁ to Z₃ are the same as the optionally substituted alkoxy groups represented by R₁ to R₁₅. A certain preferred embodiment is, for example, a phosphine compound in which Ar in compounds represented by general formula (3) are all 3,5-dimethylphenyl groups. Another preferred embodiment is, for example, a phosphine compound in which Ar in compounds represented by general formula (3) are all 4-methylphenyl groups.

In phosphite ligands represented by general formula (4), the three Y₁ may be the same or different.

The optionally substituted alkyl groups represented by Y₁ are the same as the optionally substituted alkyl groups represented by R₁ to R₁₅.

The optionally substituted aryl groups represented by Y₁ have preferably 6 to 20 carbon atoms, more preferably 6 to 14 carbon atoms, even more preferably 6 to 10 carbon atoms.

Examples of the substituents on the aryl groups represented by Y₁ include the same substituents as those of the alkyl groups represented by R₁ to R₁₅.

Examples of the optionally substituted aryl groups represented by Y₁ include phenyl groups, biphenyl groups, indenyl groups, naphthyl groups, anthryl groups, phenanthryl groups, fluorenyl groups, and pyrenyl groups; and alkyl-substituted phenyl groups such as tolyl groups, xylyl groups, trimethylphenyl groups, ethylphenyl groups, isopropylphenyl groups, and tetramethylphenyl groups.

A certain preferred embodiment is, for example, a phosphite compound in which the three Y₁ are 1,1,1,3,3,3-hexafluoro-2-propyl groups.

Another preferred embodiment is, for example, a phosphite compound in which the three Y₁ are 2,4,-di-tert-butylphenyl groups.

Examples of the monodentate phosphine compounds represented by general formula (1) include phosphine compounds having an electron donating group, such as triphenylphosphine (hereinafter, also referred to by the abbreviation "TPP"), diphenyl(o-tolyl)phosphine, tri(o-tolyl)phosphine, tri(p-tolyl)phosphine, tris(2,4,6-trimethylphenyl)phosphine, tris(2,6-dimethylphenyl)phosphine, tris(2-methoxyphenylphosphine), tris(4-methoxyphenylphosphine) (hereinafter, also referred to by the abbreviation "TMOPP"), tris(2,6-dimethoxyphenyl)phosphine (hereinafter, also referred to by the abbreviation "DMPP"), diphenyl(2-methoxyphenyl)phosphine, and 4-(dimethylamino)triphenylphosphine; and phosphine compounds having an electron withdrawing group, such as (2-fluorophenyl)diphenylphosphine, (2-chlorophenyl)diphenylphosphine, (2-bromophenyl)diphenylphosphine, (pentafluorophenyl)diphenylphosphine, bis(pentafluorophenyl)phenylphosphine (hereinafter, also referred to by the abbreviation "BPFPP"), tris(pentafluorophenyl)phosphine (hereinafter, also referred to by the abbreviation "TPFPP"), tris(4-fluorophenyl)phosphine (hereinafter, also referred to by the abbreviation "TFPP"), tris(4-chlorophenyl)phosphine, tris(4-bromophenyl)phosphine, tris(4-trifluoromethylphenyl)phosphine, tris(4-carboxyphenyl)phosphine, sodium diphenylphosphinobenzene-3-sulfonate, and trisodium triphenylphosphine-3,3',3"-trisulfonate.

Examples of the bidentate phosphine ligands represented by general formula (2) include phosphine compounds such as bis(diphenylphosphino)methane, 1,2-bis(diphenylphosphino)ethane, 1,3-bis(diphenylphosphino)propane, 1,4-bis(diphenylphosphino)butane, 1,5-bis(diphenylphosphino)pentane, 1,6-bis(diphenylphosphino)hexane, and 1,2-bis[bis(pentafluorophenyl)-phosphino]ethane.

Examples of the bidentate phosphine ligands represented by general formula (3) include (±)-2,2'-bis(diphenylphosphino)-1,1'-binaphthyl (hereinafter, also referred to by the abbreviation "BINAP"), (±)-2,2'-bis(di-p-tolylphosphino)-1,1'-binaphthyl, (±)-2,2'-bis(di-p-fluorophosphino)-1,1'-binaphthyl, (±)-2,2'-bis(di-p-trifluoromethylphosphino)-1,1'-binaphthyl, and (±)-2,2'-bis[di(3,5-xylyl)phosphino]-1,1'-binaphthyl.

Examples of the phosphite ligands represented by general formula (4) include trimethyl phosphite, triethyl phosphite, tris(1,1,1,3,3,3-hexafluoro-2-propyl)phosphite, triphenyl phosphite, and tris(2,4-di-t-butylphenyl)phosphite.

Examples of the ligands containing a nitrogen atom include compounds represented by general formula (5), compounds represented by general formula (6), and a polydentate ligand (7) containing a nitrogen-containing heterocyclic ring.

The ligands containing a nitrogen atom may be used alone, or two or more thereof may be used in combination.

R₃₆R₃₇N-X₂-NR₃₈R₃₉ (5),

wherein R₃₆ to R₃₉ each independently represent an optionally substituted alkyl group, and X₂ represents an optionally substituted divalent aliphatic group. 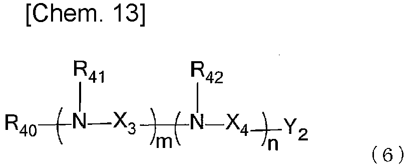
wherein R₄₀, R₄₁, and R₄₂ each independently represent an optionally substituted alkyl group, X₃ and X₄ each independently represent an optionally substituted divalent aliphatic group that may contain an oxygen atom and/or a nitrogen atom, m and n each independently represent an integer of 1 or more, Y₂ represents an optionally substituted monoalkylamino group or dialkylamino group, and any two or more of R₄₀, R₄₁, R₄₂, and Y₂ may together form a ring, and R₄₁, R₄₂, X₃, and X₄ may be the same or different when R₄₁, R₄₂, X₃, and X₄ are plural.

The optionally substituted alkyl groups represented by R₃₆ to R₃₉ are the same as the optionally substituted alkyl groups represented by R₁ to R₁₅.

The optionally substituted divalent aliphatic groups represented by X₂ are the same as the optionally substituted divalent aliphatic groups represented by X₁.

The optionally substituted alkyl groups represented by R₄₀, R₄₁, and R₄₂ are the same as the optionally substituted alkyl groups represented by R₁ to R₁₅. The number of carbon atoms in the monoalkylamino group (-NHR^{a} (R^{a} represents an alkyl group)) and dialkylamino group (-NR^{b}R^{c} (R^{b} and R^{c} represent alkyl groups)) represented by Y₂ is not particularly limited, and is preferably 1 to 12, more preferably 1 to 6, even more preferably 1 to 4, particularly preferably 1 to 3. Examples of the alkyl groups in the monoalkylamino group and dialkylamino group represented by Y₂ include the optionally substituted alkyl groups represented by R₁ to R₁₅ satisfying the foregoing carbon counts. The dialkylamino group may be one having the foregoing carbon counts in each alkyl group.

Examples of the optionally substituted monoalkylamino group represented by Y₂ include methylamino groups, ethylamino groups, propylamino groups, isopropylamino groups, butylamino groups, isobutylamino groups, t-butylamino groups, pentylamino groups, and hexylamino groups.

Examples of the optionally substituted dialkylamino group represented by Y₂ include dimethylamino groups, diethylamino groups, dipropylamino groups, diisopropylamino groups, dibutylamino groups, diisobutylamino groups, dipentylamino groups, dihexylamino groups, and ethylmethylamino groups. The alkyl groups in the monoalkylamino group and dialkylamino group represented by Y₂ may be substituted with a substituent. Examples of the substituent include the same substituents as those of the alkyl groups represented by R₁ to R₁₅.

The divalent aliphatic groups represented by X₃ and X₄ may be linear or branched. The divalent aliphatic groups have preferably 1 to 20 carbon atoms, more preferably 1 to 16 carbon atoms, even more preferably 1 to 12 carbon atoms, particularly preferably 1 to 8 carbon atoms.

Examples of the divalent aliphatic groups include alkylene groups, alkenylene groups, and alkynylene groups. Preferred are alkylene groups. Examples of the alkylene groups include dimethylpropylene groups, pentamethylene groups, hexamethylene groups, heptamethylene groups, octamethylene groups, nonamethylene groups, decamethylene groups, undecamethylene groups, and dodecamethylene groups.

Examples of the substituents on the divalent aliphatic groups represented by X₃ and X₄ include the same substituents as those of the divalent aliphatic group represented by X₁. The divalent aliphatic groups represented by X₃ and X₄ may contain an oxygen atom and/or a nitrogen atom. X₃ and X₄ may be the same or different.

The symbols m and n each independently represent an integer of 1 or more, preferably an integer of 1 to 8, more preferably an integer of 1 to 6, even more preferably an integer of 1 to 5, particularly preferably an integer of 1 to 3. The symbols m and n may be the same or different.

Any two or more of R₄₀, R₄₁, R₄₂, and Y₂ may together form a ring. For example, R₄₀, R₄₁, or R₄₂ may form a ring with Y₂. Alternatively, R₄₀ and Y₂, and Y₂ and R₄₂ may separately form a ring, creating a compound with two rings. Furthermore, the nitrogen atom in the amino group represented by Y₂ may form a ring with R₄₀. The ring may contain an oxygen atom and/or a nitrogen atom.

In certain embodiments, compounds represented by general formula (6) may be compounds having a bicyclic ring. For example, in certain embodiments, compounds represented by general formula (6) may be compounds having a bicyclic ring, with a ring formed by Y₂ and R₄₀, and another ring formed by R₄₁ and Y₂ or R₄₂ and Y₂.

In a certain preferred embodiment, R₄₀, R₄₁, and R₄₂ are linear or branched C1 to C6 alkyl groups that may have a substituent.

Another certain preferred embodiment is, for example, a dental curable composition in which the ligand compound (F) is at least one selected from the group consisting of a compound represented by general formula (1), a compound represented by general formula (4), a compound represented by general formula (5), a compound represented by general formula (6), and a polydentate ligand (7) containing a nitrogen-containing heterocyclic ring.

Yet another certain preferred embodiment is, for example, a dental curable composition in which the ligand compound (F) is a compound represented by general formula (6), wherein, in the compound represented by general formula (6), R₄₀, R₄₁, and R₄₂ represent linear or branched C1 to C6 alkyl groups that may have a substituent, the divalent aliphatic groups represented by X₃ and X₄ represent alkylene groups containing no oxygen atom and no nitrogen atom, m and n each independently represent an integer of 1 or more, Y₂ represents an optionally substituted monoalkylamino group or dialkylamino group, and Y₂ and R₄₀ together form a ring.

Still another certain preferred embodiment is, for example, a dental curable composition in which at least one of the first and second agents comprises a ligand compound (F), and the ligand compound (F) is a compound represented by general formula (6), wherein, in the general formula (6), m is 1 and n is 2, creating a compound containing four nitrogen atoms as a whole.

The polydentate ligand (7) containing a nitrogen-containing heterocyclic ring represents a bidentate or higher dentate ligand compound that comprises a heterocyclic ring containing a five-membered or six-membered ring having a nitrogen atom, and has two or more nitrogen atoms within the molecule. The polydentate ligand (7) has two or more nitrogen atoms within the molecule, and may have three or more nitrogen atoms within the molecule. The polydentate ligand (7) may contain one heterocyclic ring, or two or more heterocyclic rings.

Examples of the nitrogen-containing heterocyclic ring include nitrogen-containing five-membered rings such as a pyrrole ring, a pyrazole ring, and an imidazole ring; and nitrogen-containing six-membered rings such as a pyridine ring, a pyrazine ring, a pyridazine ring, a piperazine ring, a pyrimidine ring, and a triazine ring.

The nitrogen-containing heterocyclic ring may be a fused ring formed by the nitrogen atom-containing five-membered or six-membered ring with other rings (for example, an aromatic ring), or a fused ring formed by nitrogen atom-containing five-membered or six-membered rings.

Examples of the fused rings formed by the nitrogen atom-containing five-membered or six-membered ring with an aromatic ring include a quinoline ring, an isoquinoline ring, an indole ring, a benzoimidazole ring, and a benzotriazole ring.

The polydentate ligand (7) comprises a heterocyclic ring containing a nitrogen atom-containing five-membered or six-membered ring. An example is a ligand compound that comprises a fused ring, such as an indole ring, a benzoimidazole ring, or a benzotriazole ring, and a heterocyclic ring containing a nitrogen atom-containing five-membered or six-membered ring. Concerning denticity, the polydentate ligand (7) is a bidentate or higher dentate ligand compound, and may be tridentate or tetradentate, for example.

Examples of polydentate amine ligands represented by the general formula (5) include two-coordinate polydentate amine compounds such as N,N,N',N'-tetramethylethylenediamine (hereinafter, also referred to by the abbreviation "TMEDA"), N,N,N',N'-tetramethylpropylenediamine (hereinafter, also referred to by the abbreviation "TMPDA"), N,N,N',N'-tetramethyl-1,4-diaminobutane, N,N,N',N'-tetraethylethylenediamine (hereinafter, also referred to by the abbreviation "TEEDA"), and N,N,N',N'-tetrakis(2-hydroxyethyl)ethylenediamine.

Examples of compounds represented by the general formula (6) include polydentate amine compounds, for example, compounds having a cyclic ring, such as 1,4,8,11-tetramethyl-1,4,8,11-tetraazacyclotetradecane, compounds having a bicyclic ring such as 4,11-dimethyl-1,4,8,11-tetraazabicyclohexadecane, and compounds with no rings, such as 2,5,9,12-tetramethyl-2,5,9,12-tetraazatetradecane, 2,6,9,13-tetramethyl-2,6,9,13-tetraazatetradecane, 2,5,8,12-tetramethyl-2,5,8,12-tetraazatetradecane, N,N,N,N',N",N"-pentamethyldiethylenetriamine (hereinafter, also referred to by the abbreviation "PMDETA"), hexamethyltris(2-aminoethyl)amine, N,N-bis(2-dimethylaminoethyl)-N,N'-dimethylethylenediamine (hereinafter, also referred to by the abbreviation "HMTETA"), and tris[2-(dimethylamino)ethyl]amine (hereinafter, also referred to by the abbreviation "Me₆TREN").

Examples of the polydentate ligand (7) containing a nitrogen-containing heterocyclic ring include polydentate ligands having one nitrogen-containing heterocyclic ring, such as N-(n-propyl)pyridylmethaneimine, and N-(n-octyl)pyridylmethaneimine; and polydentate ligands having two or more nitrogen-containing heterocyclic rings, such as 2,2-bipyridine, 4,4'-di-(5-nonyl)-2,2'-bipyridine, N-propyl-N,N-di(2-pyridylmethyl)amine, N',N"-dimethyl-N',N"-bis((pyridin-2-yl)methyl)ethane-1,2-diamine, 2,6-bis(1-pyrazole)-pyridine (hereinafter, also referred to by the abbreviation "DPP"), 2-(2-pyridyl)benzoimidazole, tris[(2-pyridyl)methyl]amine, 3,6-di(2-pyridyl)-1,2,4,5-tetrazine, N,N,N',N'-tetrakis(2-pyridylmethyl)ethylenediamine, and 2,4,6-tri(2-pyridyl)-1,3,5-triazine.

Preferred for use among these are TPP, tri(o-tolyl)phosphine, DMPP, TMOPP, BPFPP, TPFPP, TFPP, BINAP, TMEDA, TMPDA, TEEDA, PMDETA, Me₆TREN, HMTETA, and DPP.

The ligand compound (F) may be incorporated alone, or two or more thereof may be incorporated in combination. The ligand compound (F) is used to enhance the catalytic activity of the chemical polymerization initiator system in a dental curable composition of the present invention.

In view of curability, the mechanical strength of the cured product, and adhesive properties to tooth structure, the content of ligand compound (F) ranges preferably from 0.001 to 10 parts by mass, more preferably from 0.005 to 8 parts by mass, even more preferably from 0.01 to 5 parts by mass, particularly preferably from 0.05 to 4 parts by mass with respect to total 100 parts by mass of polymerizable monomer components in the dental curable composition.

In view of even superior adhesive properties to tooth structure, a certain preferred embodiment is, for example, a dental curable composition in which the content of ligand compound (F) ranges from 0.005 to 2.5 parts by mass with respect to total 100 parts by mass of polymerizable monomer components in the dental curable composition when it is used as a dental cement.

In a dental curable composition of the present invention, the content of chemical polymerization initiator (C) and the content of ligand compound (F) are preferably 1:0.0001 to 1:10 in terms of a mass ratio of chemical polymerization initiator (C):ligand compound (F). In view of achieving even superior curability, enhanced mechanical strength of the cured product, and greater adhesive properties to tooth structure, the mass ratio of chemical polymerization initiator (C):ligand compound (F) is more preferably 1:0.005 to 1:5, even more preferably 1:0.01 to 1:2.5.

With the dental curable composition comprising the ligand compound (F) in predetermined proportions relative to the chemical polymerization initiator (C) within these ranges, it is possible to more greatly enhance the activity of the polymerization initiator system.

A dental curable composition of the present invention may further comprise a filler (G) to provide sufficient workability in the composition, and achieve sufficient X-ray opacity and mechanical strength in the cured product.

The filler (G) may be any type of filler, provided that it does not hinder the effectiveness of the present invention. Examples include inorganic fillers, organic fillers, and composite fillers of inorganic fillers and organic fillers.

The filler (G) may be incorporated alone, or two or more thereof may be incorporated in combination.

The filler (G) has an average particle diameter of preferably 0.001 to 10 µm, more preferably 0.001 to 5 µm.

Examples of the inorganic fillers include silica; silica-based minerals such as kaolin, clay, *ummo,* and mica; and various types of ceramic and glass containing base material silica and other components such as Al₂O₃, B₂O₃, TiO₂, ZrO₂, BaO, La₂O₃, SrO, ZnO, CaO, P₂O₅, Li₂O, and Na₂O. Examples of such glass include lithium borosilicate glass, borosilicate glass, bioglass, lanthanum glass, barium glass, strontium glass, soda glass, zinc glass, and fluoroaluminosilicate glass. Also preferred for use as inorganic fillers are crystal quartz, hydroxyapatite, alumina, titanium oxide, yttrium oxide, zirconia, barium sulfate, aluminum hydroxide, sodium fluoride, potassium fluoride, sodium monofluorophosphate, lithium fluoride, and ytterbium fluoride.

In view of adhesion and ease of handling, preferred for use is particulate silica having an average particle diameter of 0.001 to 10 µm.

A certain preferred embodiment is, for example, a dental curable composition that further comprises a filler (G), and the filler (G) comprises an inorganic filler having an average particle diameter of 0.001 µm or more and 0.1 µm or less, and an inorganic filler having an average particle diameter of more than 0.1 µm and 10 µm or less.

The inorganic fillers may be commercially available products. Examples of such commercially available products include Aerosil^{®} OX50, Aerosil^{®} 50, Aerosil^{®} 200, Aerosil^{®} 380, Aerosil^{®} R972, Aerosil^{®} 130, and AEROXIDE^{®} Alu C (all manufactured by Nippon Aerosil Co., Ltd. under these trade names).

In the present invention, the average particle diameter of inorganic filler means the average particle diameter before surface treatment when the inorganic filler is surface treated as will be described later.

Examples of the organic fillers include polymethyl methacrylate, polyethyl methacrylate, polymers of polyfunctional methacrylate, polyamides, polystyrene, polyvinyl chloride, chloroprene rubber, nitrile rubber, and styrene-butadiene rubber.

Examples of the composite fillers of inorganic fillers and organic fillers include those comprising inorganic fillers dispersed in organic fillers, and inorganic/organic composite fillers comprising various types of polymers coating the inorganic filler.

The filler (G) may be used after a surface treatment with a known surface treatment agent such as a silane coupling agent, in order to improve curability, mechanical strength, and ease of handling.

Examples of such surface treatment agents include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The average particle diameter (average primary particle diameter) can be determined by a laser diffraction scattering method or by electron microscopy of particles. Specifically, a laser diffraction scattering method is more convenient for the measurement of particles that are 0.1 µm or larger, whereas electron microscopy is a more convenient method of particle diameter measurement for ultrafine particles of less than 0.1 µm. Here, a value of 0.1 µm is the reference value, measured by a laser diffraction scattering method.

As an example of a laser diffraction scattering method, the particle size may be measured by volume using a laser diffraction particle size distribution analyzer (SALD-2300, manufactured by Shimadzu Corporation) with a 0.2% sodium hexametaphosphate aqueous solution used as dispersion medium.

A scanning electron microscope (e.g., SU3800 or S-4000 manufactured by Hitachi High-Technologies Corporation) may be used for electron microscopy. In electron microscopy, the particle size can be measured by taking an electron micrograph of particles, and the size of particles (at least 200 particles) observed in a unit field of the captured image may be measured using image-analyzing particle size distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

The content of filler (G) is not particularly limited, as long as the present invention can exhibit its effects. However, the content of filler (G) ranges preferably from 50 to 500 parts by mass, more preferably from 80 to 400 parts by mass, even more preferably from 100 to 300 parts by mass with respect to total 100 parts by mass of polymerizable monomer components in a dental curable composition of the present invention. With the content of filler (G) confined within these ranges, the cured product can have sufficient X-ray opacity or mechanical strength, and the paste can exhibit sufficient workability.

The content of filler (G) is not particularly limited, as long as the present invention can exhibit its effects. However, the content of filler (G) is preferably 50 to 97 mass%, more preferably 55 to 95 mass%, even more preferably 60 to 90 mass% in a total amount of a dental curable composition of the present invention.

A dental curable composition of the present invention comprises a redox-type polymerization initiator. However, in order to provide a dual-cure dental curable composition also capable of polymerizing upon exposure to light, or a dental kit (for example, the dental kit (Y-2) described below) comprising a dental curable composition of the present invention with a dental adhesive that undergoes polymerization upon exposure to light, a known photopolymerization initiator system (H) may additionally be incorporated in the dental curable composition, separately from the polymerization initiator system described above.

Examples of the photopolymerization initiator system (H) include α-diketones, ketals, thioxanthones, (bis)acylphosphine oxides, and α-aminoacetophenones.

Examples of the α-diketones include dl-camphorquinone (hereinafter, also referred to by the abbreviation "CQ"), benzyl, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, 2,4,6-trimethylbenzoylphenylphosphine oxide, 2,4,6-trimethylbenzoylmethoxyphenylphosphine oxide, 2,4,6-trimethylbenzoylethoxyphenylphosphine oxide, benzoylbis(2,6-dimethylphenyl)phosphine oxide, the water-soluble acylphosphine oxide compounds disclosed in JP H03-57916 B, and salts of these (for example, sodium salts, potassium salts, ammonium salts).

Examples of bisacylphosphine oxides include bis(2,4,6-trimethylbenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, dibenzoylphenylphosphine oxide, tris(2,4-dimethylbenzoyl)phosphine oxide, tris(2-methoxybenzoyl)phosphine oxide, and salts of these (for example, sodium salts, potassium salts, ammonium salts). Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the α-aminoacetophenones include 2-benzyl-2-dimethylamino-1-(4-morpholinophenyl)-1-butanone, 2-benzyl-2-diethylamino-1-(4-morpholinophenyl)-1 -butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

For enhanced photocurability, the photopolymerization initiator may be used with polymerization accelerators for use with photopolymerization initiators. Examples of polymerization accelerators for photopolymerization initiators include tertiary amines, aldehydes, benzaldehyde derivatives, thiol compounds, and triazine compounds substituted with a trihalomethyl group.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, 2-(methacryloyloxy)ethyl 4-(N,N-dimethylamino)benzoate, ethyl 4-(N,N-dimethylamino)benzoate, butyl 4-(N,N-dimethylamino)benzoate, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethyl (meth)acrylate, N-methyldiethanolamine di(meth)acrylate, N-ethyldiethanolamine di(meth)acrylate, triethanolamine mono(meth)acrylate, triethanolamine di(meth)acrylate, and triethanolamine tri(meth)acrylate.

Examples of the aldehydes include terephthalaldehyde, and benzaldehyde derivatives.

Examples of the benzaldehyde derivatives include dimethylaminobenzaldehyde, p-methoxybenzaldehyde, p-ethoxybenzaldehyde, and p-n-octyloxybenzaldehyde.

Examples of the thiol compounds include 3-mercaptopropyltrimethoxysilane, 2-mercaptobenzoxazole, 2-mercaptobenzoimidazole, decanethiol, and thiobenzoic acid.

The triazine compounds substituted with a trihalomethyl group may be any known compounds, provided that it is an s-triazine compound with at least one trihalomethyl group such as a trichloromethyl group and a tribromomethyl group.

Individually, the photopolymerization initiators, and the polymerization accelerators for photopolymerization initiators may be used alone, or two or more thereof may be used in combination.

The content of photopolymerization initiators, and the content of polymerization accelerators for photopolymerization initiators are not particularly limited. However, in view of properties such as the curability of the dental cement obtained, the content is preferably 0.001 to 10 parts by mass, more preferably 0.005 to 5 parts by mass, even more preferably 0.01 to 3 parts by mass with respect to total 100 parts by mass of the polymerizable monomer components.

A dental curable composition of the present invention may additionally comprise a fluorine-ion releasing substance. By incorporating a fluorine-ion releasing substance, a dental cement can be obtained that can impart acid resistance to tooth structure.

Examples of the fluorine-ion releasing substance include fluorine-ion releasing polymers, such as copolymers of methyl methacrylate and methacrylic acid fluoride; hydrofluorides of primary, secondary, or tertiary amines of aliphatic or alicyclic nature, such as cetylamine hydrofluoride, cyclohexylamine hydrofluoride, diisobutylamine hydrofluoride, and triethylamine trihydrofluoride; and metal fluorides such as sodium fluoride, potassium fluoride, sodium monofluorophosphate, lithium fluoride, and ytterbium fluoride.

The fluorine-ion releasing substance may be used alone, or two or more thereof may be used in combination.

In a dental curable composition of the present invention, it is also possible to incorporate additives such as pH adjusters, polymerization inhibitors, ultraviolet absorbers, solvents (such as organic solvents, and water), thickeners, colorants, antimicrobial agents, and fragrances, provided that such additives do not hinder the effectiveness of the present invention. These may be used alone, or two or more thereof may be used in combination.

Examples of the pH adjusters include the tertiary amines exemplified above as polymerization accelerators for photopolymerization initiators.

Examples of the organic solvents include alcohols (for example, such as ethanol, methanol, and isopropanol), ethers, and ketones (for example, such as acetone, and methyl ethyl ketone).

A dental curable composition of the present invention can be prepared according to an ordinary method, depending on the aforementioned components. A dental curable composition of the present invention can be implemented in various forms, appropriately selected from, for example, powder and liquid, paste and liquid, two pastes and liquid, and two pastes.

In view of workability, more preferred embodiments are paste and liquid, two pastes and liquid, and two pastes.

When using two pastes, each paste is preferably kept isolated from the other during storage, and the two pastes are kneaded immediately before use to initiate chemical polymerization for curing. Usually, the pastes are prepared by mixing a liquid component formulated with components other than filler (G), and kneading it with the filler (G) (powder). When used in either two-paste and liquid form or in the form of paste and liquid, the composition is configured in a way that allows chemical polymerization to take place at the interface of the liquid and the paste when these come into contact with each other.

A certain preferred embodiment (X) is, for example, a two-paste dental curable composition comprising a first agent and a second agent.

In the two-paste dental curable composition, it is preferable that the first agent comprise a polymerizable monomer (A) having an acidic group, a polymerizable monomer (B) having no acidic group, and a chemical polymerization initiator (C), and the second agent comprise a polymerizable monomer (B) having no acidic group, and a polymerization accelerator (D), and that the first or second agent comprise a transition metal compound (E) and a ligand compound (F).

A certain preferred embodiment (X-1) is, for example, a dental curable composition in which the first agent comprises a transition metal compound (E), and the second agent comprises a ligand compound (F).

Another preferred embodiment (X-2) is, for example, a dental curable composition in which the first agent comprises a transition metal compound (E) and a ligand compound (F).

Yet another preferred embodiment (X-3) is, for example, a dental curable composition in which the first agent comprises a ligand compound (F), and the second agent comprises a transition metal compound (E).

Still another preferred embodiment (X-4) is, for example, a dental curable composition in which the second agent comprises a transition metal compound (E) and a ligand compound (F).

The flexural strength of cured products of a dental curable composition of the present invention is not particularly limited, as long as the desired flexural strength can be achieved according to intended use. For example, when the dental curable composition is used as a material for constructing abutments, the flexural strength is preferably 80 MPa or more, more preferably 85 MPa or more, even more preferably 90 MPa or more. For example, when the dental curable composition is used for dental resin cements (preferably, self-adhesive dental resin cements), the flexural strength is preferably 20 MPa or more, more preferably 25 MPa or more, even more preferably 30 MPa or more. The method of measurement of flexural strength is as described in the EXAMPLES section below.

Another certain embodiment (X-5) is, for example, a dental curable composition in which the polymerization accelerator (D) in any of the aforementioned two-paste dental curable compositions is at least one selected from the group consisting of an aromatic amine, an aromatic sulfinic acid and salts thereof, a sulfur-containing reducing inorganic compound, a thiourea compound, an ascorbic acid compound, a borate compound, and a barbituric acid compound.

Although favorable adhesive properties to tooth structure are achieved in Patent Literature 4 as a dental curable composition, factors other than the catalyst system contribute significantly to the adhesive properties. One such factor is the high total content of HEMA (hydrophilic polymerizable monomer (B-1)) and MDP (polymerizable monomer (A) having an acidic group), as noted in the EXAMPLES section. However, a potential consequence of such high content of these components is increased water absorbency, possibly leading to reduced mechanical strength durability. It was also observed that chemical curability needs further improvement with a low total content of hydrophilic polymerizable monomer (B-1) and polymerizable monomer (A) having an acidic group. In contrast, a dental curable composition of the present invention, by containing the ligand compound (F), can achieve favorable adhesive properties to tooth structure while reducing a decrease in the durability of mechanical strength, even when the total content of polymerizable monomer (A) having an acidic group and hydrophilic polymerizable monomer (B-1) is less than 20 parts by mass in total 100 parts by mass of polymerizable monomer components in the dental curable composition.

In a dental curable composition of the present invention, the total content of polymerizable monomer (A) having an acidic group and hydrophilic polymerizable monomer (B-1) may be 18 parts or less by mass in total 100 parts by mass of polymerizable monomer components in the dental curable composition.

Because a dental curable composition of the present invention can also greatly enhance the activity of the polymerization initiator system, and achieve outstanding mechanical strength durability by comprising the ligand compound (F), the total content of polymerizable monomer (A) having an acidic group and hydrophilic polymerizable monomer (B-1) may be 20 parts or more by mass in total 100 parts by mass of polymerizable monomer components in the dental curable composition. By comprising the ligand compound (F), a dental curable composition of the present invention can achieve more favorable adhesive properties to tooth structure compared to Patent Literature 4, even when the total content of polymerizable monomer (A) having an acidic group and hydrophilic polymerizable monomer (B-1) is 20 parts or more by mass in total 100 parts by mass of polymerizable monomer components in the dental curable composition.

A dental curable composition of the present invention may be a dual-cure dental curable composition. Another certain embodiment (X-6) is, for example, a dental curable composition in which the polymerization accelerator (D) in any of the aforementioned two-paste dental curable compositions comprises at least one selected from the group consisting of a benzotriazole compound or benzoimidazole compound, an aromatic amine, a salt of aromatic sulfinic acids, a sulfur-containing reducing inorganic compound, a thiourea compound, and an ascorbic acid compound, and that comprises a photopolymerization initiator system (H).

In any of the embodiments of the two-paste dental curable compositions described above (including embodiment (X-1) to embodiment (X-6)), the type and amount of each component may be varied as appropriate according to the foregoing descriptions, and changes such as addition and deletion can be made to any component. For example, in the embodiments above, desired proportions can be set for the mass ratio between the content of transition metal compound (E) and the content of ligand compound (F), and/or the mass ratio between the content of chemical polymerization initiator (C) and the content of ligand compound (F).

In any of the embodiments above, various properties (such as the flexural strength, flexural modulus, and working time margin of the cured product) can be adjusted to a suitable range according to intended use by appropriately varying the formulation of each dental curable composition.

Aside from the embodiments involving a single agent (for example, the two-paste dental curable composition), the present invention also encompasses embodiments involving a dental kit comprising the components of the dental curable composition described above. Descriptions of embodiments of such dental kits are provided below.

Within each dental kit, the ligand compound (F) is included, and the ligand compound (F) is combined with other components such as the polymerization initiator system, as in a dental curable composition of the present invention. This enables the present invention to exhibit its effects by greatly enhancing the activity of the polymerization initiator system. Moreover, the other components do not interfere with the activity of the polymerization initiator system. Hence, the embodiments below, including dental kit (Y-1) and dental kit (Y-2), also represent variations of the present invention, aside from the two-paste dental curable compositions described earlier. That is, the application of the present invention is not restricted and can extend to various dental applications. The type and content of each component used in a dental curable composition of the present invention are also applicable to each dental kit, unless otherwise specifically stated.

A certain preferred embodiment of the present invention is, for example, a dental kit (Y-1) comprising a water-based dental adhesive composition (I) and a dental paste composition (II) (hereinafter, also referred to as "paste-form dental curable composition (II)"), wherein:
the water-based dental adhesive composition (I) comprises a polymerizable monomer (A) having an acidic group, a ligand compound (F), and water, and
the dental paste composition (II) comprises a polymerizable monomer (B) having no acidic group, a chemical polymerization initiator (C), a polymerization accelerator (D), a transition metal compound (E), and a filler (G).

The content of the polymerizable monomer (A) having an acidic group in the water-based dental adhesive composition (I) is preferably 3 to 35 parts by mass, more preferably 5 to 30 parts by mass, even more preferably 10 to 25 parts by mass in total 100 parts by mass of the liquid components of the water-based dental adhesive composition (I).

In view of the ability to greatly enhance the activity of the polymerization initiator system in the dental kit, as well as curability, the mechanical strength of the cured product, and adhesive properties to tooth structure, the content of the ligand compound (F) in the water-based dental adhesive composition (I) is preferably 0.1 to 10 parts by mass, more preferably 0.3 to 8 parts by mass, even more preferably 0.5 to 5 parts by mass in total 100 parts by mass of the liquid components of the water-based dental adhesive composition (I).

The water content in the water-based dental adhesive composition (I) is preferably 15 to 60 parts by mass, more preferably 18 to 55 parts by mass, even more preferably 20 to 50 parts by mass in total 100 parts by mass of the liquid components of the water-based dental adhesive composition (I). The water-based dental adhesive composition (I) may comprise a solvent (for example, organic solvent) other than water.

In this specification, examples of the liquid components that can be contained in the water-based dental adhesive composition (I) include a polymerizable monomer (A) having an acidic group, a polymerizable monomer (B) having no acidic group, and a solvent (such as water, or organic solvents).

The dental paste composition (II) in dental kit (Y-1) may or may not comprise the ligand compound (F). The type and content of each component in the dental paste composition (II) are the same as in a dental curable composition of the present invention.

Another certain preferred embodiment of the present invention is, for example, a dental kit (Y-2) comprising a dental adhesive composition (III) and a dental paste composition (II), wherein:
the dental adhesive composition (III) comprises a polymerizable monomer (A) having an acidic group, a polymerizable monomer (B) having no acidic group, a ligand compound (F), and a photopolymerization initiator system (H), and
the dental paste composition (II) comprises a polymerizable monomer (B) having no acidic group, a chemical polymerization initiator (C), a polymerization accelerator (D), a transition metal compound (E), and a filler (G).

The content of the polymerizable monomer (A) having an acidic group in the dental adhesive composition (III) is preferably 1 to 50 parts by mass, more preferably 1 to 45 parts by mass, even more preferably 2 to 40 parts by mass in 100 parts by mass of the liquid components of the dental adhesive composition (III).

The content of the polymerizable monomer (B) having no acidic group in the dental adhesive composition (III) is preferably 40 to 99 parts by mass, more preferably 45 to 98 parts by mass, even more preferably 50 to 98 parts by mass in 100 parts by mass of the liquid components of the dental adhesive composition (III).

In view of the ability to greatly enhance the activity of the polymerization initiator system in the dental kit, as well as curability, the mechanical strength of the cured product, and adhesive properties to tooth structure, the content of the ligand compound (F) in the dental adhesive composition (III) is preferably 0.1 to 10 parts by mass, more preferably 0.3 to 8 parts by mass, even more preferably 0.5 to 5 parts by mass in 100 parts by mass of the liquid components of the dental adhesive composition (III).

The content of the photopolymerization initiator system (H) in the dental adhesive composition (III) is preferably 0.001 to 20 parts by mass, more preferably 0.005 to 15 parts by mass, even more preferably 0.01 to 10 parts by mass in total 100 parts by mass of polymerizable monomer components in the dental adhesive composition (III).

The dental paste composition (II) in dental kit (Y-2) may or may not comprise the ligand compound (F). The type and content of each component in the dental paste composition (II) are the same as in a dental curable composition of the present invention.

A dental curable composition of the present invention has use in bonding dental prostheses such as crowns, inlays, and bridges to tooth structures, as well as in the construction of abutments in defects in the affected tooth area.

A dental curable composition of the present invention can be used as a dental cement such as a dental resin cement. A dental curable composition of the present invention is particularly suited for use as a dental resin cement, a self-adhesive dental resin cement, or an abutment construction material.

The present invention encompasses embodiments combining the foregoing features in various ways within the technical idea of the present invention, provided that the present invention can exhibit its effects.

### EXAMPLES

The following describes the present invention in greater detail using Examples and Comparative Examples. It is to be noted, however, that the present invention is not limited to the following. The abbreviations and symbols used hereafter are as follows. The compounds and fillers used in the following Examples and Comparative Examples are commercially available products, except where the production methods are specifically described.

### [Polymerizable monomer (A) having an acidic group]

MDP: 10-methacryloyloxydecyl dihydrogen phosphate

### [Polymerizable monomer (B) containing no acidic group]

HEMA: 2-hydroxyethyl methacrylate
Bis-GMA: 2,2-bis[4-(2-hydroxy-3-methacryloyloxypropoxy)phenyl]propane
D2.6E: 2,2-bis(4-methacryloyloxypolyethoxyphenyl)propane (with an average of 2.6 moles of ethoxy group added)
TEGDMA: triethylene glycol dimethacrylate
#801: 1,2-bis(3-methacryloyloxy-2-hydroxypropoxy)ethane

### [Chemical polymerization initiator (C)]

THP: 1,1,3,3-tetramethylbutyl hydroperoxide
BPB: t-butyl peroxybenzoate
KPS: potassium peroxydisulfate

### [Polymerization accelerator (D)]

DEPT: N,N-bis(2-hydroxyethyl)-p-toluidine
TPSS: 2,4,6-triisopropylbenzenesulfinic acid
Sodium sulfite: Na₂SO₃
BTA: 1H-benzotriazole
PyTU: 1-(2-pyridyl)-2-thiourea
DMETU: 4,4-dimethylethylenethiourea
PA: ascorbyl palmitate

### [Transition metal compound (E)]

CuA: copper(II) acetate
VOAA: vanadyl(IV) acetylacetonate

### [Ligand compound (F)]

TPP: triphenylphosphine
BPFPP: bis(pentafluorophenyl)phenylphosphine
TPFPP: tris(pentafluorophenyl)phosphine
TFPP: tris(4-fluorophenyl)phosphine
DMPP: tris(2,6-dimethoxyphenyl)phosphine
TMOPP: tris(4-methoxyphenylphosphine)
BINAP: (±)-2,2'-bis(diphenylphosphino)-1,1'-binaphthyl
PMDETA: N,N,N,N',N",N"-pentamethyldiethylenetriamine
TEEDA: N,N,N',N'-tetraethylethylenediamine
Me₅TREN: tris[2-(dimethylamino)ethyl]amine

### [Filler (G)]

### Surface-treated Ba glass powder

A barium glass (manufactured by Esstech, product code E-3000) was pulverized with a ball mill to obtain a barium glass powder. The pulverized barium glass powder had an average particle diameter of 2.4 µm as measured by volume with a laser diffraction particle size distribution analyzer (Model SALD-2300, manufactured by Shimadzu Corporation). One-hundred parts by mass of the pulverized barium glass powder was surface treated with three parts by mass of γ-methacryloyloxypropyltrimethoxysilane by an ordinary method to obtain a surface-treated Ba glass powder.
R972: particulate silica manufactured by Nippon Aerosil Co., Ltd. under the trade name Aerosil^{®} R972; average particle diameter: 16 nm
Alumina: aluminum oxide manufactured by Nippon Aerosil Co., Ltd. under the trade name AEROXIDE^{®} Alu C; average particle diameter: 13 nm

### [Photopolymerization initiator system (H)]

CQ: dl-camphorquinone

### [Polymerization accelerator for photopolymerization initiators]

PDE: ethyl 4-(N,N-dimethylamino)benzoate

### [pH Adjuster]

DMAEMA: 2-(dimethylamino)ethyl methacrylate
TTA: triethanolamine

### [Polymerization inhibitor]

BHT: 2,6-di-t-butyl-4-methylphenol

### Examples 1-1 to 1-21 and Comparative Examples 1-1 to 1-3)

The components, excluding the fillers, listed in Tables 1 and 2 were mixed at ordinary temperature to prepare a uniform liquid component. Subsequently, this liquid component was kneaded with the fillers to prepare dental curable compositions of Examples 1-1 to 1-21 and Comparative Examples 1-1 to 1-3.

These dental curable compositions were used to measure 23°C working time, bond strength to bovine dentin, and flexural strength and flexural modulus following the methods described below. Tables 1 and 2 present the formulation ratios (parts by mass) of the dental curable compositions, along with the test results.

**[Table 1]**

| | Components (parts by mass) | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 | Ex. 1-11 | Ex. 1-12 | Ex. 1-13 | Ex. 1-14 |
|---|---|---|---|---|---|---|---|---|---|---|---|---|---|---|---|---|
| First agent | Acidic group-containing polymerizable monomer (A) | MDP | 15 | 15 | 15 | 15 | 15 | 15 | 15 | 15 | 15 | 15 | 20 | 15 | 15 | 15 |
| | Polymerizable monomer (B) having no acidic group | HEMA | 10 | 10 | 10 | 10 | 10 | 10 | 10 | 10 | 10 | 10 | 20 | 10 | 10 | 10 |
| | | Bis-GMA | 20 | 20 | 20 | 20 | 20 | 20 | 20 | 20 | 20 | 20 | 40 | 20 | 20 | 20 |
| | | D2.6E | 30 | 30 | 30 | 30 | 30 | 30 | 30 | 30 | 30 | 30 | 20 | 30 | 30 | 30 |
| | | TEGDMA | 25 | 25 | 25 | 25 | 25 | 25 | 25 | 25 | 25 | 25 | | 25 | 25 | 25 |
| | Chemical polymerization initiator (C) | THP | | | | | | | | | | | | 2 | 2 | 2 |
| | | BPB | 1 | 1 | 1 | 1 | 1 | 1 | 1 | 1 | 1 | 1 | 1 | | | |
| | | KPS | 1 | 1 | 1 | 1 | 1 | 1 | 1 | 1 | 1 | 1 | 1 | | | |
| | Transition metal compound (E) | CuA | 0.0007 | 0.0007 | 0.0007 | 0.0007 | 0.0007 | 0.0007 | 0.0007 | 0.0007 | 0.0007 | 0.0007 | 0.0007 | 0.005 | 0.005 | 0.005 |
| | Ligand compound (F) | PMDETA | | | | | | | | | | | | | | |
| | | Me6TREN | | | | | | | | | | | | | | |
| | | TEEDA | | | | | | | | | | | | | | |
| | Polymerization inhibitor | BHT | 0.15 | 0.15 | 0.15 | 0.15 | 0.15 | 0.15 | 0.15 | 0.15 | 0.15 | 0.15 | 0.15 | 0.15 | 0.15 | 0.15 |
| | Filler (G) | Surface-treated Ba class powder | 200 | 200 | 200 | 200 | 200 | 200 | 200 | 200 | 200 | 200 | 200 | 200 | 200 | 200 |
| | | R972 | 30 | 30 | 30 | 30 | 30 | 30 | 30 | 30 | 30 | 30 | 30 | 30 | 30 | 30 |
| Second agent | Polymerizable monomer (B) having no acidic group | D2.6E | 85 | 85 | 85 | 85 | 85 | 85 | 85 | 85 | 85 | 85 | 85 | 85 | 85 | 85 |
| | | TEGDMA | 15 | 15 | 15 | 15 | 15 | 15 | 15 | 15 | 15 | 15 | 15 | 15 | 15 | 15 |
| | Polymerization accelerator (D) | DEPT | 0.3 | 0.3 | 0.3 | 0.3 | 0.3 | 0.3 | 0.3 | 0.3 | 0.3 | 0.3 | 0.3 | | | |
| | | TPSS | 2.5 | 2.5 | 2.5 | 2.5 | 2.5 | 2.5 | 2.5 | 2.5 | 2.5 | 2.5 | 2.5 | | | |
| | | Sodium sulfite | 3 | 3 | 3 | 3 | 3 | 3 | 3 | 3 | 3 | 3 | 3 | | | |
| | | BTA | 3 | 3 | 3 | 3 | 3 | 3 | 3 | 3 | 3 | 3 | 3 | | | |
| | | PyTU | | | | | | | | | | | | 0.7 | 0.7 | 0.7 |
| | | PA | | | | | | | | | | | | | | |
| | Transition metal compound (E) | VOAA | | | | | | | | | | | | | | |
| | Ligand compound (F) | TPP | | | | | | | | | | | | | | |
| | | BPFPP | 2 | 0.009 | 0.02 | 4 | 55 | | | | | | 2 | 2 | | |
| | | TPFPP | | | | | | | | | | | | | 2 | |
| | | TFPP | | | | | | | | | | | | | | 2 |
| | | DMPP | | | | | | 2 | | | | | | | | |
| | | TMOPP | | | | | | | 2 | | | | | | | |
| | | BINAP | | | | | | | | 2 | | | | | | |
| | | PMDETA | | | | | | | | | 2 | | | | | |
| | | TEEDA | | | | | | | | | | 2 | | | | |
| | Polymerization inhibitor | BHT | 0.05 | 0.05 | 0.05 | 0.05 | 0.05 | 0.05 | 0.05 | 0.05 | 0.05 | 0.05 | 0.05 | 0.05 | 0.05 | 0.05 |
| | Filler (G) | Surface-treated Ba class powder | 200 | 200 | 200 | 200 | 200 | 200 | 200 | 200 | 200 | 200 | 200 | 200 | 200 | 200 |
| | | Alumina | 30 | 30 | 30 | 30 | 30 | 30 | 30 | 30 | 30 | 30 | 30 | 30 | 30 | 30 |

| | | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 | Ex. 1-11 | Ex. 1-12 | Ex. 1-13 | Ex. 1-14 |
|---|---|---|---|---|---|---|---|---|---|---|---|---|---|---|---|---|
| Performance | 23°C Workina time (min) | | 3.5 | 3.9 | 3.7 | 2.2 | 2.1 | 2.2 | 2.1 | 3.8 | 2.2 | 2.3 | 3.4 | 2.5 | 2.9 | 3.0 |
| | Tensile bond strength to dentin (MPa) (37°C, after 1 day) | | 9.8 | 7.3 | 9.5 | 9.7 | 7.4 | 9.7 | 9.8 | 10.5 | 100 | 10.9 | 14.3 | 9.5 | 10.9 | 10.5 |
| | Flexural strenath (MPa) | | 102.1 | 95.0 | 102.1 | 107.4 | 103.2 | 101.0 | 108.9 | 106.0 | 104.4 | 105.6 | 101.3 | 115.2 | 113.0 | 105.0 |
| | Flexural modulus (GPa) | | 6.3 | 6.0 | 6.4 | 6.8 | 6.7 | 6.5 | 6.7 | 6.0 | 6.2 | 6.6 | 6.1 | 6.8 | 6.8 | 6.3 |
| | Flexural strenath (MPa) (50°C, after 28 days) | | 115.2 | 100.1 | 118.8 | 119.5 | 110.0 | 112.1 | 116.3 | 115.7 | 116.2 | 113.0 | 107.2 | 123.6 | 120.2 | 110.4 |
| | Flexural modulus (GPa) (50°C, after 28 days) | | 7.3 | 6.8 | 6.9 | 7.7 | 7.5 | 7.3 | 7.5 | 6.9 | 7.0 | 7.3 | 6.5 | 7.4 | 7.3 | 7.0 |

**[Table 2]**

| | Components (parts by mass) | | Ex. 1-15 | Ex. 1-16 | Ex. 1-17 | Ex. 1-18 | Ex. 1-19 | Ex. 1-20 | Ex. 1-21 | Com. Ex. 1-1 | Com. Ex. 1-2 | Com. Ex. 1-3 |
|---|---|---|---|---|---|---|---|---|---|---|---|---|
| First agent | Acidic group-containing polymerizable monomer (A) | MDP | 15 | 15 | 15 | 15 | 15 | 15 | 15 | 15 | 15 | 15 |
| | Polymerizable monomer (B) having no acidic group | HEMA | 10 | 10 | 10 | 10 | 10 | 10 | 10 | 10 | 10 | 10 |
| | | Bis-GMA | 20 | 20 | 20 | 20 | 20 | 20 | 20 | 20 | 20 | 20 |
| | | D2.6E | 30 | 30 | 30 | 30 | 30 | 30 | 30 | 30 | 30 | 30 |
| | | TEGDMA | 25 | 25 | 25 | 25 | 25 | 25 | 25 | 25 | 25 | 25 |
| | Chemical polymerization initiator (C) | THP | 2 | 2 | 2 | 3 | 3 | 3 | 3 | | 2 | 3 |
| | | BPB | | | | | | | | 1 | | |
| | | KPS | | | | | | | | 1 | | |
| | Transition metal compound (E) | CuA | 0.005 | 0.005 | | 0.01 | 0.01 | 0.01 | 0.01 | 0.0015 | 0.01 | 002 |
| | Ligand compound (F) | PMDETA | 2 | | | | 2 | | | | | |
| | | Me6TREN | | | | | | 2 | | | | |
| | | TEEDA | | 2 | 2 | | | | 2 | | | |
| | Polymerization inhibitor | BHT | 0.15 | 0.15 | 0.15 | 0.15 | 0.15 | 0.15 | 0.15 | 0.15 | 0.15 | 0.15 |
| | Filler (G) | Surface-treated Ba class powder | 200 | 200 | 200 | 200 | 200 | 200 | 200 | 200 | 200 | 200 |
| | | R972 | 30 | 30 | 30 | 30 | 30 | 30 | 30 | 30 | 30 | 30 |
| Second agent | Polymerizable monomer (B) having no acidic group | D2.6E | 85 | 85 | 85 | 85 | 85 | 85 | 85 | 85 | 85 | 85 |
| | | TEGDMA | 15 | 15 | 15 | 15 | 15 | 15 | 15 | 15 | 15 | 15 |
| | Polymerization accelerator (D) | DEPT | | | | | | | | 0.3 | | |
| | | TPSS | | | | | | | | 2.5 | | |
| | | Sodium sulfite | | | | | | | | 3 | | |
| | | BTA | | | | | | | | 3 | | |
| | | PyTU | 0.7 | 0.7 | 0.7 | | | | | | 0.7 | |
| | | PA | | | | 2 | 2 | 2 | 2 | | | 2 |
| | Transition metal compound (E) | VOAA | | | 0.05 | | | | | | | |
| | Ligand compound (F) | TPP | | | | 2 | | | | | | |
| | | BPFPP | | | | | | | | | | |
| | | TPFPP | | | | | | | | | | |
| | | TFPP | | | | | | | | | | |
| | | DMPP | | | | | | | | | | |
| | | TMOPP | | | | | | | | | | |
| | | BINAP | | | | | | | | | | |
| | | PMDETA | | | | | | | | | | |
| | | TEEDA | | | | | | | | | | |
| | Polymerization inhibitor | BHT | 0.05 | 0.05 | 0.05 | 0.05 | 0.05 | 0.05 | 0.05 | 0.05 | 0.05 | 0.05 |
| | Filler (G) | Surface-treated Ba class powder | 200 | 200 | 200 | 200 | 200 | 200 | 200 | 200 | 200 | 200 |
| | | Alumina | 30 | 30 | 30 | 30 | 30 | 30 | 30 | 30 | 30 | 30 |

| | | | Ex. 1-15 | Ex. 1-16 | Ex. 1-17 | Ex. 1-18 | Ex. 1-19 | Ex. 1-20 | Ex. 1-21 | Com. Ex. 1-1 | Com. Ex. 1-2 | Com. Ex. 1-3 |
|---|---|---|---|---|---|---|---|---|---|---|---|---|
| Performance | 23°C Working time (min) | | 2.0 | 2.1 | 2.5 | 4.0 | 3.3 | 3.0 | 3.1 | 4.1 | 3.5 | 80 |
| | Tensile bond strength to dentin (37°C, after 1 day) (MPa) | | 9.5 | 9.9 | 10.1 | 12.4 | 11.9 | 12.0 | 12.3 | 5.8 | 6.5 | 7.5 |
| | Flexural strength (MPa) | | 108.9 | 109.0 | 112.7 | 105.0 | 104.2 | 108.9 | 105.4 | 85.0 | 85.0 | 83.0 |
| | Flexural modulus (GPa) | | 6.3 | 6.2 | 6.4 | 6.3 | 6.4 | 6.4 | 6.4 | 5.0 | 5.2 | 4.5 |
| | Durability of flexural strength (MPa) (50°C, after 28 days) | | 114.2 | 117.8 | 117.3 | 112.4 | 111.1 | 115.6 | 113.2 | 83.2 | 84.9 | 82.5 |
| | Durability of flexural modulus (GPa) (50°C, after 28 days) | | 6.8 | 6.8 | 6.9 | 6.8 | 6.9 | 6.9 | 6.8 | 4.9 | 5 | 4.3 |

### (Production Examples A to C)

The components, excluding the fillers, listed in Table 3 were mixed at ordinary temperature to prepare a uniform liquid component. Subsequently, this liquid component was kneaded with the fillers to prepare dental paste compositions (II) of Production Examples A to C.

**[Table 3]**

| Components (parts by mass) | | | Production Example A | Production Example B | Production Example C |
|---|---|---|---|---|---|
| First agent | Acidic group-containing polymerizable monomer (A) | MDP | 15 | | 15 |
| | Polymerizable monomer (B) having no acidic group | HEMA | 10 | 10 | 10 |
| | | Bis-GMA | 20 | 20 | 20 |
| | | D2.6E | 30 | 30 | 30 |
| | | TEGDMA | 25 | 40 | 25 |
| | Chemical polymerization initiator (C) | THP | | 3 | 3 |
| | | BPB | 1 | | |
| | | KPS | 1 | | |
| | Transition metal compound (E) | CuA | 0.001 | | 0.01 |
| | Polymerization inhibitor | BHT | 0.15 | 0.15 | 0.15 |
| | Filler (G) | Surface-treated Ba glass powder | 200 | 200 | 200 |
| | | R972 | 30 | 30 | 30 |
| Second agent | Polymerizable monomer (B) having no acidic group | D2.6E | 85 | 75 | 85 |
| | | HEMA | | 10 | |
| | | TEGDMA | 15 | 15 | 15 |
| | Transition metal compound (E) | VOAA | | 0.15 | |
| | Polymerization accelerator (D) | DEPT | 0.3 | | |
| | | TPSS | 2.5 | | |
| | | Sodium sulfite | 3 | | |
| | | BTA | 3 | | |
| | | DMETU | | 4 | |
| | | PA | | | 4 |
| | Polymerization inhibitor | BHT | 0.05 | 0.05 | 0.05 |
| | Filler (G) | Surface-treated Ba glass powder | 200 | 200 | 200 |
| | | Alumina | 30 | 30 | 30 |

### (Production Examples a to k, and m and n)

The components listed in Table 4 were mixed at ordinary temperature to prepare a uniform liquid composition. Production Examples a to e represent dental adhesive compositions (III), whereas Production Examples f to k are water-based dental adhesive compositions (I). Production Example m constitutes a dental adhesive composition; however, it does not correspond to dental adhesive composition (III) due to the absence of the ligand compound (F). Production Example n constitutes a water-based dental adhesive composition but does not correspond to water-based dental adhesive composition (I) due to the absence of the ligand compound (F).

**[Table 4]**

| Components (parts by mass) | | Production Example a | Production Example b | Production Example c | Production Example d | Production Example e | Production Example f |
|---|---|---|---|---|---|---|---|
| Acidic group-containing polymerizable monomer (A) | MDP | 10 | 10 | 10 | 10 | 10 | 15 |
| Polymerizable monomer (B) having no acidic group | Bis-GMA | 30 | 30 | 30 | 30 | 30 | |
| | HEMA | 30 | 30 | 30 | 30 | 30 | 30 |
| | #801 | | | | | | 25 |
| Solvent | Water | 15 | 15 | 15 | 15 | 15 | 30 |
| | Ethanol | 15 | 15 | 15 | 15 | 15 | |
| Photopolymerization initiator system (H) | CQ | 2 | 2 | 2 | 2 | 2 | |
| Polymerization accelerator for photopolymerization initiators | PDE | 2 | 2 | 2 | 2 | 2 | |
| pH Adjuster | DMAEMA | | | | | | 2.5 |
| | TTA | 1.5 | 1.5 | 1.5 | | | |
| Transition metal compound (E) | VOAA | | | | | | |
| | CuA | | | | | | |
| Ligand compound (F) | BPFPP | 1.5 | | | | | 2.5 |
| | TFPP | | 1.5 | | | | |
| | DMPP | | | 1.5 | | | |
| | PMDETA | | | | 1.5 | | |
| | TEEDA | | | | | 1.5 | |
| Polymerization inhibitor | BHT | 0.05 | 0.05 | 0.05 | 0.05 | 0.05 | 0.05 |
| Filler (G) | R972 | 10 | 10 | 10 | 10 | 10 | |

| Components (parts by mass) | | Production Example g | Production Example h | Production Example i | Production Example j | Production Example k | Production Example m | Production Example n |
|---|---|---|---|---|---|---|---|---|
| Acidic group-containing polymerizable monomer (A) | MDP | 15 | 15 | 15 | 15 | 15 | 10 | 15 |
| Polymerizable monomer (B) having no acidic group | Bis-GMA | | | | | | 30 | |
| | HEMA | 30 | 30 | 30 | 30 | 30 | 30 | 30 |
| | #801 | 25 | 25 | 25 | 25 | 25 | | 25 |
| Solvent | Water | 30 | 30 | 30 | 30 | 30 | 15 | 30 |
| | Ethanol | | | | | | 15 | |
| Photopolymerization initiator system (H) | CQ | | | | | | 2 | |
| Polymerization accelerator for photopolymerization initiators | PDE | | | | | | 2 | |
| pH Adjuster | DMAEMA | 2.5 | 2.5 | | | | | 2.5 |
| | TTA | | | | | | 1.5 | |
| Transition metal compound (E) | VOAA | 0.3 | 0.3 | 0.3 | | 0.3 | 0.3 | 0.7 |
| | CuA | | | | 0.02 | | | |
| Ligand compound (F) | BPFPP | 2.5 | | | | | | |
| | TFPP | | | | | | | |
| | DMPP | | 2.5 | | | | | |
| | PMDETA | | | 2.5 | 2.5 | | | |
| | TEEDA | | | | | 2.5 | | |
| Polymerization inhibitor | BHT | 0.05 | 0.05 | 0.05 | 0.05 | 0.05 | 0.05 | 0.05 |
| Filler (G) | R972 | | | | | | 10 | |

### (Examples 2-1 to 2-26, Comparative Examples 2-1 to 2-2)

The paste-form dental curable composition (II) acquired from Table 3 was combined with either the dental adhesive composition or water-based dental adhesive composition obtained from Table 4. These were then measured for working time margin upon contact, and tensile bond strength to dentin following the test methods described below. Tables 5 to 7 present the pairings of the paste-form dental curable composition (II) with either the dental adhesive composition or water-based dental adhesive composition, along with the test results.

**[Table 5]**

| | | Ex. 2-1 | Ex. 2-2 | Ex. 2-3 | Ex. 2-4 | Ex. 2-5 | Ex. 2-6 | Ex. 2-7 | Ex. 2-8 | Com. Ex. 2-1 |
|---|---|---|---|---|---|---|---|---|---|---|
| Paste | | Production Example A | | | | | | | | Production Example A |
| Dental adhesive composition or water-based dental adhesive composition | | Production Example a | Production Example c | Production Example d | Production Example e | Production Example f | Production Example h | Production Example j | Production Example k | Production Example m |
| Performance | Working time margin upon contact (sec) | >120 | >120 | >120 | >120 | 110 | 80 | 100 | 80 | Fail: Solidification of dental adhesive composition |
| | Tensile bond strength to dentin (MPa) | 20.0 | 23.8 | 23.1 | 22.4 | 21.2 | 24.6 | 25.5 | 25.1 | Fail: Solidification of dental adhesive composition |

**[Table 6]**

| | | Ex. 2-9 | Ex. 2-10 | Ex. 2-11 | Ex. 2-12 | Ex. 2-13 | Ex. 2-14 | Ex. 2-15 | Ex. 2-16 | Ex. 2-17 | Com. Ex. 2-2 |
|---|---|---|---|---|---|---|---|---|---|---|---|
| Paste | | Production Example B | | | | | | | | | Production Example B |
| Dental adhesive composition or water-based dental adhesive composition | | Production Example a | Production Example b | Production Example d | Production Example e | Production Example f | Production Example g | Production Example i | Production Example j | Production Example k | Production Example n |
| Performance | Working time margin upon contact (sec) | >120 | >120 | >120 | >120 | 110 | 60 | 70 | 80 | 70 | Unmeasurable |
| | Tensile bond strength to dentin (MPa) | 21.9 | 22.9 | 23.6 | 24.0 | 20.9 | 24.6 | 24.3 | 23.9 | 25.1 | 26.5 |

**[Table 7]**

| | | Ex. 2-18 | Ex. 2-19 | Ex. 2-20 | Ex. 2-21 | Ex. 2-22 | Ex. 2-23 | Ex. 2-24 | Ex. 2-25 | Ex. 2-26 |
|---|---|---|---|---|---|---|---|---|---|---|
| Paste | | Production Example C | | | | | | | | |
| Dental adhesive composition or water-based dental adhesive composition | | Production Example a | Production Example c | Production Example d | Production Example e | Production Example f | Production Example g | Production Example i | Production Example j | Production Example k |
| Performance | Working time margin upon contact (sec) | >120 | >120 | >120 | >120 | 110 | 90 | 90 | 100 | 90 |
| | Tensile bond strength to dentin (MPa) | 21.0 | 23.2 | 23.6 | 24.0 | 20.1 | 24.5 | 23.1 | 25.0 | 24.2 |

### [23°C Working Time of Dental Curable Composition]

The first agent and second agent were mixed at a 1:1 mass ratio within a thermostatic chamber set at 23°C. These were thoroughly mixed with a spatula to form a single agent. Using a thermocouple (manufactured by Okazaki Manufacturing Company) linked to a recorder (manufactured by Yokogawa Electric Corporation), the duration from the moment of mixing until the temperature began to rise due to the onset of paste's curing (referred to as working time) was measured. The working time represents the average of measured values from five test samples.

A working time ranging from 2 to 8 minutes is considered suitable for practical use. For more practicality, a range between 2 minutes and under 5 minutes is more preferred.

### [Bond Strength of Dental Curable Composition to Bovine Dentin]

The labial surface of a bovine mandibular incisor was ground with #80 silicon carbide paper (manufactured by Nihon Kenshi Co., Ltd.) under running water to expose a flat dentin surface. The exposed flat surface was then polished with #1000 silicon carbide paper (manufactured by Nihon Kenshi Co., Ltd.) under running water. After polishing, the surface was dried by blowing air and removing the surface water. After drying, an about 150 µm-thick adhesive tape having a 3 mm round hole was attached to the dried smooth dentin surface to define a bonding area. The test was conducted as follows.

### (Examples 1-1 to 1-21 and Comparative Examples 1-1 to 1-3)

The first agent and second agent from each dental curable composition were taken in equal amounts. These were kneaded for a duration of 10 seconds, and the resulting mixture was applied onto one end surface (with a circular cross section) of a cylindrical stainless steel rod (7 mm in diameter, 2.5 cm in length).

Subsequently, the end surface with the dental curable composition was placed on the smooth surface (adherend) within the round hole, with the center of the round hole approximately aligned with the center of the cylindrical stainless steel rod. The cylindrical stainless steel rod was then pressed perpendicularly against the smooth surface to effect bonding and prepare a test sample. A total of five test samples were prepared. These test samples were left at 25°C for 30 minutes, and immersed in distilled water. The test samples were then left for 24 hours in the distilled water, inside a thermostatic chamber held at 37°C. After this 24-hour period at 37°C, the test samples were measured for their tensile bond strength.

The tensile bond strength was measured using a universal testing machine (Autograph AG-I 100kN, manufactured by Shimadzu Corporation) with the crosshead speed set at 2 mm/min. The table presents the tensile bond strength as an average of measured values from five test samples after a 24-hour period at 37°C.

### (Examples 2-1 to 2-26 and Comparative Examples 2-1 to 2-2)

The dental adhesive compositions or water-based dental adhesive compositions prepared in Production Examples a to k were applied to the smooth surface (adherend) within the round hole. In Production Examples a to e, the applied surface was left to stand for 3 seconds before blowing air to dry the dental adhesive composition until it was no longer flowable. Similarly, in Production Examples f to k, the applied surface was left to stand for 20 seconds before blowing air to dry the water-based dental adhesive composition until it was no longer flowable.

Subsequently, the first agent and second agent from each paste-form dental curable composition (II) of Production Examples A to C were taken in equal amounts. These were kneaded for a duration of 10 seconds, and the resulting mixture was applied onto one end surface (with a circular cross section) of a cylindrical stainless steel rod (7 mm in diameter, 2.5 cm in length). The end surface with the dental curable composition was then placed on the smooth surface (adherend) within the round hole, with the center of the cylindrical stainless steel rod approximately aligned with the center of the round hole treated with the liquid formulation. The cylindrical stainless steel rod was then pressed perpendicularly against the smooth surface to effect bonding and prepare a test sample. A total of five test samples were prepared. These test samples were left at 25°C for 30 minutes, and immersed in distilled water. The test samples were then left for 24 hours in the distilled water, inside a thermostatic chamber held at 37°C. After this 24-hour period at 37°C, the test samples were measured for their tensile bond strength.

The tensile bond strength was measured using a universal testing machine (Autograph AG-I 100kN, manufactured by Shimadzu Corporation) with the crosshead speed set at 2 mm/min. The table presents the tensile bond strength as an average of measured values from five test samples after a 24-hour period at 37°C.

### [Flexural Strength and Flexural Modulus of Cured Product of Dental Curable Composition]

A polyester film was laid over a glass slide, and a stainless-steel mold, measuring 2 mm in length, 25 mm in width, and 2 mm in depth, was mounted on the film.

Subsequently, a mixture of the first and second agents of the dental curable composition of the present invention was filled into the mold. The composition was then compressed within the mold by pressing another glass slide against the surface of the composition via another polyester film, and the glass slides were secured with 25 mm-wide binder clips. With the binder clips securing the sample, the sample was left to undergo polymerization and curing for 1 hour in a 37°C thermostatic chamber, and the resulting cured composition was removed from the mold after taking the sample out of the thermostatic chamber. For storage, the cured product was immersed in 37°C distilled water for 24 hours, and the flexure test was conducted using this specimen.

The flexural strength and flexural modulus were measured by a three-point flexural test carried out with a span of 20 mm and a crosshead speed of 1 mm/min, using a universal testing machine (AG-I 100kN, manufactured by Shimadzu Corporation). The mean value of flexural strengths and the mean value of flexural moduli from five specimens were determined as the flexural strength and flexural modulus of each sample.

### [Durability of Flexural Strength and Flexural Modulus of Cured Product of Dental Curable Composition]

A polyester film was laid over a glass slide, and a stainless-steel mold, measuring 2 mm in length, 25 mm in width, and 2 mm in depth, was mounted on the film.

Subsequently, a mixture of the first and second agents of the dental curable composition of the present invention was filled into the mold. The composition was then compressed within the mold by pressing another glass slide against the surface of the composition via another polyester film, and the glass slides were secured with 25 mm-wide binder clips. With the binder clips securing the sample, the sample was left to undergo polymerization and curing for 1 hour in a 37°C thermostatic chamber, and the resulting cured composition was removed from the mold after taking the sample out of the thermostatic chamber. For storage, the cured product was immersed in 37°C distilled water for 24 hours, followed by immersion in 50°C water for 28 days. The flexure test was conducted using this specimen.

The flexural strength and flexural modulus were measured by a three-point flexural test carried out with a span length (span) of 20 mm and a crosshead speed of 1 mm/min, using a universal testing machine (AG-I 100kN, manufactured by Shimadzu Corporation).

The mean value of flexural strengths and the mean value of flexural moduli from five specimens were determined as the durability of flexural strength and the durability of flexural modulus of each sample.

### [Working Time Margin upon Contact]

The labial surface of a bovine mandibular incisor was ground with #80 silicon carbide paper (manufactured by Nihon Kenshi Co., Ltd.) under running water to obtain a sample with an exposed flat dentin surface. The flat surface was large enough to sufficiently accommodate the base of a 9 mm diameter cylinder. The sample was then immersed in distilled water after polishing the flat surface with #1000 silicon carbide paper (manufactured by Nihon Kenshi Co., Ltd.). Subsequently, a cylindrical SUS tip (9 mm in diameter, approximately 7 mm in height) was polished with #1000 silicon carbide paper (manufactured by Nihon Kenshi Co., Ltd.) over a circular cross section.

The bovine tooth, immersed in distilled water, and the polished SUS tip prepared in this fashion were used for the test after being left to stand for 2 hours in a thermostatic chamber that had been set to 35°C.

Following the placement of the bovine tooth in the thermostatic chamber, the surface was dried by blowing air and removing water. The tooth was then secured onto a glass slide using Utility Wax (manufactured by GC JAPAN), with the smooth surface facing upward. Subsequently, the glass slide holding the bovine tooth was situated on a work surface within the thermostatic chamber. The positioning of the bovine tooth was adjusted so that its smooth surface remained parallel to the work surface.

Thereafter, the dental adhesive composition or water-based dental adhesive composition was applied to the smooth surface with a brush. In Production Examples a to e, the applied surface was dried by blowing after 3 seconds of settling from application, allowing the dental adhesive composition to dry until it was no longer flowable. Similarly, in Production Examples f to k, the applied surface was dried by blowing after 20 seconds of settling from application, allowing the water-based dental adhesive composition to dry until it was no longer flowable.

This was followed by application of a mixture of the first and second agents of the paste-form dental curable composition (II) from Production Examples A to C onto the polished surface of the SUS tip. This SUS tip was then delicately placed on the bovine tooth to ensure contact between its composition applied surface and the smooth surface of the bovine tooth. Immediately after contact, a 150-g cylindrical weight (3 cm in diameter) was placed on the SUS tip and then removed after 1 second. The measurement commenced upon removal of the weight.

After 30 seconds from the start of measurement, the SUS tip was gently toughed with a finger to assess any movement. This action was repeated every 10 seconds, and the moment at which the SUS tip became unmovable was determined as the point marking the working time margin upon contact. It is desirable to extend the working time margin upon contact as long as possible, and the practical working time margin upon contact is 50 seconds or longer.

As shown in Tables 1 and 2, the dental curable compositions according to the present invention (Examples 1-1 to 1-21) demonstrated an adequate working time at 23°C, and produced outstanding results in bond strength to bovine dentin, and in both flexural strength and flexural modulus.

The dental curable compositions of Comparative Examples 1-1 to 1-3 lacking the ligand compound (F) exhibited an adequate working time at 23°C. However, these compositions failed to show other properties, and were inferior in results compared to Examples 1-1 to 1-21.

Furthermore, Comparative Examples 1-1 to 1-3 with no ligand compound (F) were unable to show the same level of properties as in Examples, despite an increase in the content of transition metal compound (E) to increase the overall content of the chemical polymerization initiator system with the anticipation of improving chemical curability, as is evident from the comparison between, for example, Examples 1-1 to 1-6 and Comparative Example 1-1.

In the present invention, even with the lower content of transition metal compound (E) in comparison to Comparative Examples, it was confirmed that the coordination of the ligand compound (F) to the metal atom of the transition metal compound (E) enhanced the catalytic activity of the chemical polymerization initiator system, achieving outstanding bond strength to bovine dentin, as well as producing excellent flexural strength and flexural modulus, while ensuring the desired working time.

As indicated in Tables 5 to 7, the dental curable compositions according to the present invention (Examples 2-1 to 2-26) presented no practical issue in working time margin upon contact between the dental adhesive composition or water-based dental adhesive composition and the paste-form composition, and demonstrated outstanding results in terms of bond strength to bovine dentin.

In Comparative Example 2-1 employing Production Example m with no ligand compound (F), a vanadium compound was incorporated as a transition metal compound in the dental adhesive composition (Production Example m) in place of the ligand compound (F) to provide bond strength. This resulted in solidification following adjustments, rendering the composition unusable, as shown in Table 5.

In Comparative Example 2-2 employing Production Example n with no ligand compound (F), a high concentration of transition metal compound was incorporated as a substitute for the ligand compound (F) in the water-based dental adhesive composition (Production Example n) to provide high bond strength. However, despite achieving a high bond strength to bovine dentin, the working time margin upon contact was too brief to be measured, indicating that the composition is impractical, as shown in Table 6.

### INDUSTRIAL APPLICABILITY

A dental curable composition of the present invention can be suitably used in applications such as luting of dental prostheses, such as crowns, inlays, and bridges, to tooth structures, and construction of abutments in dental treatments.

## Claims

1. A dental curable composition comprising a polymerizable monomer (A) having an acidic group, a polymerizable monomer (B) having no acidic group, a chemical polymerization initiator (C), a polymerization accelerator (D), a transition metal compound (E), and a ligand compound (F), wherein the ligand compound (F) is at least one compound selected from the group consisting of a ligand containing a phosphorus atom, and a ligand containing a nitrogen atom.

2. The dental curable composition according to claim 1, wherein:
the ligand compound (F) is a ligand containing a phosphorus atom, and
the ligand containing a phosphorus atom is at least one compound selected from the group consisting of a compound represented by the following general formula (1), a compound represented by general formula (2), a compound represented by general formula (3), and a compound represented by general formula (4), 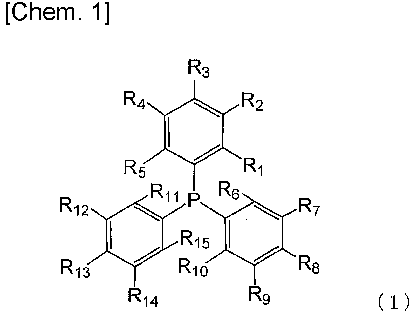 wherein R₁ to R₁₅ each independently represent a hydrogen atom, a halogen atom, a polar group, an optionally substituted alkyl group, or an optionally substituted alkoxy group, 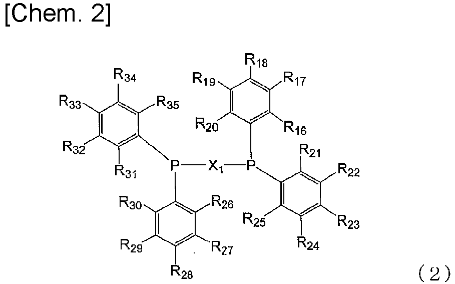 wherein R₁₆ to R₃₅ each independently represent a hydrogen atom, a halogen atom, a polar group, an optionally substituted alkyl group, or an optionally substituted alkoxy group, and X₁ represents an optionally substituted divalent aliphatic group, 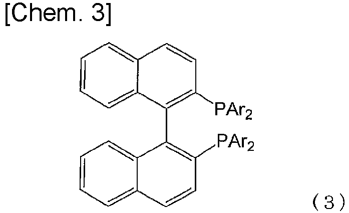 wherein Ar each independently represent a group represented by the following general formula (3-a), 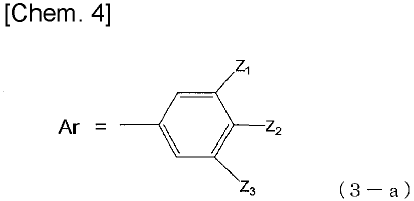 wherein Z₁ to Z₃ are each independently a hydrogen atom, a halogen atom, an optionally substituted alkyl group, or an optionally substituted alkoxy group, and at least one of Z₁ to Z₃ is a hydrogen atom,
P(OY₁)₃ (4),
wherein Y₁ each independently represent an optionally substituted alkyl group, or an optionally substituted aryl group.

3. The dental curable composition according to claim 2, wherein the ligand containing a phosphorus atom is at least one compound selected from the group consisting of a compound represented by general formula (1), and a compound represented by general formula (2).

4. The dental curable composition according to claim 1, wherein:
the ligand compound (F) is a ligand containing a nitrogen atom,
the ligand containing a nitrogen atom is at least one compound selected from the group consisting of a compound represented by general formula (5), a compound represented by general formula (6), and a polydentate ligand (7) containing a nitrogen-containing heterocyclic ring, and
the polydentate ligand (7) is a bidentate or higher dentate ligand compound that comprises a heterocyclic ring containing a five-membered or six-membered ring having a nitrogen atom, and has two or more nitrogen atoms within the molecule,
R₃₆R₃₇N-X₂-NR₃₈R₃₉ (5),
wherein R₃₆ to R₃₉ each independently represent an optionally substituted alkyl group, and X₂ represents an optionally substituted divalent aliphatic group, 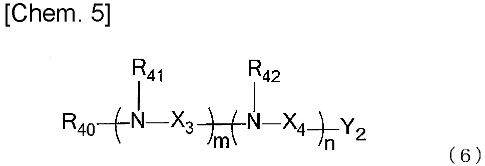 wherein R₄₀, R₄₁, and R₄₂ each independently represent an optionally substituted alkyl group, X₃ and X₄ each independently represent an optionally substituted divalent aliphatic group that may contain an oxygen atom and/or a nitrogen atom, m and n each independently represent an integer of 1 or more, Y₂ represents an optionally substituted monoalkylamino group or dialkylamino group, and any two or more of R₄₀, R₄₁, R₄₂, and Y₂ may together form a ring, and R₄₁, R₄₂, X₃, and X₄ may be the same or different when R₄₁, R₄₂, X₃, and X₄ are plural.

5. The dental curable composition according to any one of claims 1 to 4, wherein the chemical polymerization initiator (C) is at least one selected from the group consisting of a hydroperoxide, a peroxyester, and a peroxydisulfate.

6. The dental curable composition according to any one of claims 1 to 5, wherein the polymerization accelerator (D) is at least one selected from the group consisting of a thiourea compound, an aromatic sulfinic acid and a salt thereof, and an ascorbic acid compound.

7. The dental curable composition according to any one of claims 1 to 6, wherein the transition metal compound (E) is a copper compound and/or a vanadium compound.

8. The dental curable composition according to any one of claims 1 to 7, which further comprises a filler (G).

9. The dental curable composition according to any one of claims 1 to 8, which is a two-paste composition comprising a first agent and a second agent.

10. The dental curable composition according to claim 9, wherein: the first agent comprises the polymerizable monomer (A) having an acidic group, the polymerizable monomer (B) having no acidic group, and the chemical polymerization initiator (C),
the second agent comprises the polymerizable monomer (B) having no acidic group, and the polymerization accelerator (D), and
the first agent or the second agent comprises the transition metal compound (E) and the ligand compound (F).

11. The dental curable composition according to any one of claims 1 to 10, wherein the content of the ligand compound (F) is 0.01 to 5 parts by mass with respect to total 100 parts by mass of the polymerizable monomer components in the dental curable composition.

12. The dental curable composition according to any one of claims 1 to 11, wherein the content of the chemical polymerization initiator (C) and the content of the ligand compound (F) are 1:0.0001 to 1:10 in terms of a mass ratio of chemical polymerization initiator (C):ligand compound (F).

13. A dental kit comprising a water-based dental adhesive composition (I) and a dental paste composition (II), wherein:
the water-based dental adhesive composition (I) comprises a polymerizable monomer (A) having an acidic group, a ligand compound (F), and water, and
the dental paste composition (II) comprises a polymerizable monomer (B) having no acidic group, a chemical polymerization initiator (C), a polymerization accelerator (D), a transition metal compound (E), and a filler (G).

14. A dental kit comprising a dental adhesive composition (III) and a dental paste composition (II), wherein:
the dental adhesive composition (III) comprises a polymerizable monomer (A) having an acidic group, a polymerizable monomer (B) having no acidic group, a ligand compound (F), and a photopolymerization initiator system (H), and
the dental paste composition (II) comprises a polymerizable monomer (B) having no acidic group, a chemical polymerization initiator (C), a polymerization accelerator (D), a transition metal compound (E), and a filler (G).
